# EUROPEAN PATENT SPECIFICATION

(11) **EP 4 190 173 B1**
(45) Date of publication and mention of the grant of the patent: **17.07.2024**
(21) Application number: 20800703.9
(22) Date of filing: 23.09.2020
(51) Int. Cl.: A61K 45/06, A23L 33/115, A23L 33/125, A23L 33/15, A23L 33/16, A23L 33/175, A23L 33/00, A61K 31/195, A61P 35/02, A61P 35/04, A61P 35/00

(54) **METABOLIC CANCER THERAPY**
STOFFWECHSEL-KREBSTHERAPIE
THÉRAPIE METABOLIQUE CONTRE LE CANCER

(43) Date of publication of application: 07.06.2023
(73) Proprietor: AMINOVITA S.L., 41013 Sevilla (ES); Universidad De Sevilla, 41004 Sevilla (ES)
(72) Inventor: LÓPEZ-LÁZARO, Miguel, 41012 Sevilla (ES); CALDERÓN-MONTAÑO, José Manuel, 41012 Sevilla (ES); JIMÉNEZ-ALONSO, Julio José, 41012 Sevilla (ES); GUILLÉN-MANCINA, Emilio, 41012 Sevilla (ES); JIMÉNEZ-GONZÁLEZ, Víctor, 41012 Sevilla (ES); MATE-BARRERO, Alfonso, 41012 Sevilla (ES); PÉREZ-GUERRERO, María Concepción, 41012 Sevilla (ES); BURGOS-MORÓN, Estefanía, 41012 Sevilla (ES)
(74) Representative: Carlos Hernando, Borja
(86) International application number: PCT/ES2020/070571
(87) International publication number: WO 2022/064079

(56) References cited:
- WO-A1-2020/047361
- US-A1- 2003 129 262
- US-A1- 2012 082 760
- US-A1- 2014 112 909
- US-A1- 2014 227 373
- KILL KIMBERLY A.: "Unproven Cancer Therapies", AMERICAN PHARMACY., vol. 28, no. 2, 1 February 1988 (1988-02-01), pages 18 - 22, XP055805519, ISSN: 0160-3450, Retrieved from the Internet <URL:http://dx.doi.org/10.1016/S0160-3450(15)32027-4> DOI: 10.1016/S0160-3450(15)32027-4

## Description

### TECHNICAL FIELD OF THE INVENTION

The present invention is directed to an artificial diet composition for use in the treatment of cancer which is characterized by comprising specific amino acids in controlled amounts, present in the composition either in the free, salt, ester form and/or provided through an amino acid source such as a protein, said artificial diet composition comprising additionally carbohydrates and lipids plus other ingredients, such as vitamins, minerals, choline and optionally a water based and/or a pharmaceutical carrier. The artificial diet composition of the invention comprises, (based on the total weight of the dry ingredients composition):
- from 4 to 40% of a mixture of amino acids,
- from 0 - 25% lipids,
- from 40 - 95% carbohydrates,
- from 1 to 5% of a mixture of vitamins and minerals, and
- from 0 to 1% choline,
characterized in that Leucine is present in the composition as part of the mixture of amino acids in an amount of from 0,5 -6% by weight with respect to the total weight of the dry ingredients composition and Methionine is present in the composition as part of the mixture of amino acids in an amount of from 0,1 - 0,6% by weight with respect to the total weight of the dry ingredient composition.

The composition of the present invention has shown to be effective for the treatment of cancer in subjects, such as mammals, as confirmed by the experimental work showing increased survival rate of mice treated with said artificial diet compositions.

### BACKGROUND OF THE INVENTION

Pharmacotherapy is the standard of care for patients with metastasis. When the disease is spread and surgery and radiotherapy are no longer curative, drug therapy becomes the main form of treatment. Pharmacotherapy can prolong patients' lives and palliate some disease-related symptoms. However, it does not usually cure the disease. The low efficacy of the existing anticancer drugs is reflected in the poor survival rates of patients diagnosed with the most common metastatic cancers. The five-year relative survival rates for patients with distant metastasis are 5% in lung cancer, 31% in prostate cancer, 27% in breast cancer, 14% in colorectal cancer, 25% in melanoma, 12% in renal cancer, 29% in ovarian cancer, 16% in cancers of the uterine corpus, 17% in cancers of the uterine cervix, 5% in bladder cancer, 5% in esophageal cancer, 2% in liver cancer, and 3% in pancreatic cancer _{[1]}. Many patients with metastasis do not overcome the disease despite surviving five years after diagnosis.

Understanding why pharmacotherapy usually fails is important to develop better therapies. When one treats cancer cells with specific concentrations of approved anticancer drugs and examines the cells under the microscope, one generally observes a massacre. All cancer cells die in response to most treatments. However, these same drugs cannot save the lives of cancer patients. The main reason is that these drugs have a limited selectivity towards cancer cells. The consequence of this narrow selectivity is that patients cannot receive the drug doses required to kill all their cancer cells; such doses would also kill their normal body cells and would be lethal. As an alternative, they receive the maximum tolerated doses, which are usually insufficient to reach the drug concentrations required to eradicate their cancer cells. The surviving cancer cells continue to proliferate in an uncontrolled way until they eventually lead to a fatal outcome [2].

Pharmacotherapy also fails because some cancer cells are or become resistant to the drugs _{[3,4]}. The most common reason for resistance is the expression of ATP-binding cassette (ABC) efflux transporters, which eject anticancer drugs from cells. These transporters are expressed in normal stem cells under physiological conditions; these cells have to remain intact for the entire life of an organism and need powerful defense mechanisms against environmental chemical insults. Recent evidence strongly suggests that cancer arises from normal stem cells _{[5-7]}. After accumulating enough DNA alterations, normal stem cells give rise to cancer stem cells (CSCs) _{[5-7]}, which keep on expressing ABC transporters _{[8,9]}. CSCs probably eject the drugs through these transporters and resist therapy. This suggests that even if we developed more selective anticancer drugs, mechanisms that have evolved to protect cells against chemical insults from the environment would continue to act as obstacles to successful treatment of cancer _{[3]}.

Cancer pharmacotherapy can also fail because most drugs preferentially target rapidly dividing cells. Resting and slow-proliferating cancer cells, such as CSCs, usually resist therapy. In addition, some resting and slow-proliferating cancer cells are located in poorly vascularized tumor areas. Since the anticancer drugs are delivered to the cells through the blood, tumor cells located in these areas will be exposed to lower drug concentrations than normal cells (which have an adequate blood supply). This factor reduces the already limited selectivity of the existing anticancer drugs and contributes to therapy failure.

Improving the outcome of patients with metastasis requires the development of therapies with a high selectivity towards cancer cells. In addition, these therapies should overcome the drug-resistance mechanisms of these cells. They should also be effective against non-dividing cancer cells and poorly vascularized tumor cells.

The main limitation of cancer pharmacotherapy is its low selectivity towards cancer cells. With the discovery of CSCs, it has often been assumed that the main limitation of the existing treatments is their inability to kill CSCs _{[10]}. Evidence has accumulated that pharmacotherapy is ineffective at killing CSCs. However, this does not mean that the existing drugs can selectively kill the rest of cancer cells. As discussed elsewhere, the problem for most cancers is not that a few cancer cells survive treatment, but that only a few cancer cells die in response to treatment _{[11]}. Successful cancer therapy requires the development of therapies with a high selectivity towards all types of cancer cells.

The basis for developing selective anticancer therapies is similar to that for developing selective anti-infective treatments. The aim is to eliminate the infectious agent or the cancer cells without harming the patient too much. The way is to find major and exploitable differences between our cells and the infectious agent, or between our normal cells and the cancer cells.

There exists a major difference between normal cells and all types of cancer cells: unlike normal cells, cancer cells have an extremely altered DNA. As explained elsewhere _{[12]}, if one looks at most tumor cells, it looks like someone set off a bomb in the nucleus. There are big pieces of chromosomes hooked together and gains and losses of whole chromosomes in most tumor cells _{[12,13]}. The karyotype of some tumor cells is strikingly different from that of normal cells; for example, some studies have reported malignant cells with over 100 chromosomes (http://cgap.nci.nih.gov/Chromosomes/Mitelman). Within chromosomes, thousands of DNA mutations and epigenetic alterations are present in many tumors _{[14-16]}. Using whole genome sequencing information from 22086 cancer samples, a recent study showed that the mean and median number of mutations in genes (which represents less than 2% of the whole DNA) were, respectively, 177 and 61 _{[16]}. It is actually surprising that cells with so many DNA alterations are able to survive.

Current therapies do not fully exploit this major difference between cancer cells and normal cells. The new drugs are usually designed to target single DNA defects of malignant cells. For example, cancer cells commonly have mutations in genes encoding particular protein kinases. Because these proteins play an important role in cancer cell proliferation, many of the drugs recently approved for cancer therapy have been designed to inhibit specific kinases. However, exploiting minor differences between cancer cells and normal cells usually leads to minor improvements in patient survival. It has been estimated that the recent approval of 71 anticancer drugs has only led to a median overall survival benefit of 2.1 months, balanced against an estimated 10,000 dollars per month on therapy at a cost of 2.7 million dollars per life year saved _{[17-20]}. Current trends suggest that successful therapy of a particular cancer may require finding drugs for each of the driving mutations of that cancer. Given the complexity and variability of the cancer genome, the clinical benefit of this strategy may be limited _{[16, 21,22]}.

The key to developing highly selective anticancer therapies probably lies on finding a way to exploit the complete set of DNA alterations of cancer cells, and this can be achieved by creating a challenging cellular environment that only cells with undamaged DNAs can overcome. Normal cells would use their intact DNA to activate genetic and epigenetic programs to adapt to and survive the new conditions. Cancer cells, however, may be unable to survive in the new environment. The activation of these adaptation programs may require the expression of genes that, in cancer cells, may be lost, mutated or silenced. Some of these genes may be in chromosomes or pieces of chromosomes that were lost during carcinogenesis. Others may be mutated and non-functional. In addition, the activation of a genetic program may require changes in other programs that cancer cells may need to keep unchanged for survival. A challenging cellular environment can be created without drugs. Because surgery and radiation therapy cannot eliminate non-localized tumor cells, it is often assumed that drug therapy is the only possible way to successfully treat patients with metastasis. By entering the bloodstream, a drug can potentially reach and kill any non-localized cancer cell. Although cancer cells can be killed by administering a cytotoxic agent, they can also be killed by restricting something they need to survive. The result seems to be the same; however, targeting cancer cells without drugs may overcome many drug-resistance mechanisms of cancer cells (e.g., there are no drugs to pump out of the cells through ABC transporters). In addition, the location of cancer cells in poorly vascularized tumor areas may not compromise the efficacy of a restriction therapy.

Selective killing of cancer cells by amino acid restriction is one approach taken to combat cancer. The state of the art has made several attempts to solve this problem by providing protein-free artificial diets in which the levels of particular amino acids are eliminated or restricted.

WO 2017/144877 describes a dietary product for use in the treatment of cancer comprising a plurality of amino acids, which comprises all the essential amino acids and is devoid of at least two non-essential amino acids selected from the group consisting of: glycine, serine, cysteine, tyrosine and arginine. Suitable combinations of non-essential amino acids not present in the dietary compositions are: glycine, serine and cysteine; glycine, serine and arginine; glycine, serine and tyrosine; glycine, serine, arginine and cysteine; glycine, serine, tyrosine and cysteine; cysteine and arginine; cysteine and tyrosine; Cysteine and glycine; Cysteine, tyrosine and arginine; or glycine, serine, arginine, tyrosine and cysteine. Further, the dietary product may further comprise methionine at a level of less than 25mg/kg body weight of the subject/day or less than 20mg/kg/day or less than 18mg/kg/day or less than 16mg/kg/day.

WO2017/053328 describes methods of treating cancer by identifying nutritional weaknesses of cancer cells and using nutritional therapy to suppress cancer by putting a subject on a diet that deprives cancerous cells of a nutrient needed for cancer proliferation and growth. The invention also describes that such nutritional therapy can be used to enhance the effectiveness of current cancer treatments. In one embodiment of the invention described in this patent application, the amino acid-containing supplement does not contain cysteine or cystine, thereby reducing the patient's daily intake of said amino acids from 70 -100%.

US2013/0330419 refers to a dietary composition for a patient with a tumor, which includes depleted or reduced amino acid concentrations of at least 50% reduction from normal consumption of at least one amino acid selected from the grop consisting of: arginine, glutamine, methionine, asparagine, phenylalanine, histidine, glycine, tryptophan, leucine, threonine, valine, cystine, isoleucine, lysine, aspartic acid and tyrosine. In particular, the invention contemplates a dietary composition useful for the treatment of breast cancer, wherein the dietary composition includes depleted or reduced amino acid concentrations of at least one of: Arg, Gln, Asn, Phe, and His.

When the tumor is associated with prostate cancer, depleted or reduced amino acid concentration is with respect to Gin, Gly, Trp, Arg, Leu, His and Met. When the tumor is associated with lung cancer, depleted or reduced amino acid concentration is with respect to His, Gln, Asn, Cys, Leu, Met and Trp. When the tumor is associated with colorectal cancer, depleted or reduced amino acid concentration is with respect to Thr, Gly, Met, Cys, Phe, Tyr, Trp, Asn and Val. When the tumor is associated with head and neck cancer, depleted or reduced amino acid concentration is with respect to Met, Cys, Tyr, Leu and Asp.

EP1572093 discloses methods of preventing various conditions, in particular, cancer and conditions associated with cancer treatment, including metastasis by administration of glutamine or a pharmaceutically acceptable salt thereof. In a preferred embodiment this patent discloses coadministration of glutamine and an effcetive amount of a carbohydrate, such as a saccharide.

US2003/129262 is primarily directed to a dietary method of treating cancer with compositions which induce Methionine reducing levels in the subject. Compositions used in the treatment comprise the commercial diet products, named Hominex^{®}-2 and "Tumorex", where Hominex^{®}- 2 contains 0.0 g Methionine, and "Tumorex" contains 0.03 g Methionine per 100g of powder product.

US 2014/112909 discloses methods for tissue and/or organ regeneration. In one example a low calorie fasting-mimicking diet (FMD) is disclosed to achieve reduction in cancer incidence, delay the onset of cancer-related death and/or affect biomarkers associated with health- and lifespan in subjects.

US2012/082760 is based upon the insight that inhibiting the mTOR pathway can have beneficial effects for health and therefore this document refers to producing generally nutritionally complete protein supplements, where levels of Leucine and/or Methionine are reduced to zero or near zero, and in embodiments are intended for cancer patients.

Despite the efforts made in this technical field, there still exists the need in the state of the art of providing alternative artificial diet compositions which are effective for the treatment of cancer and/or show enhanced anti-cancer activity with respect to the pharmacological treatments used in patients with cancer.

The present invention is therefore faced with the problem of providing an effective anti-cancer artificial diet composition as well as methods of using the same.

### BRIEF DESCRIPTION OF THE INVENTION

The following disclosure is presented to provide an illustration of the general principles of the present invention and is not meant to limit, in any way, the inventive concepts contained herein.

All terms defined herein should be afforded their broadest possible interpretation, including any implied meanings.

It should be stated that, as recited herein, the singular forms "a", "an", and "the" include the plural referents unless otherwise stated. Additionally, the terms "comprises" and "comprising" when used herein specify that certain features are present in that embodiment, however, this phrase should not be interpreted to preclude the presence or addition of additional steps, operations, features and/or components.

As used in the present invention, the term "dry composition" comprises all the ingredients in the artificial diet composition of the present invention except water, for example, amino acid mixtures, proteins, carbohydrates, lipids, choline, vitamins and minerals.

The terms "subject" or "patient" are used interchangeably herein and refer to a vertebrate, preferably a mammal. Mammals include, but are not limited to, humans.

As used herein, the terms "treating," "treatment" and the like are used herein, without limitation, to mean obtaining a desired pharmacologic and/or physiologic effect. The effect may be prophylactic in terms of completely or partially preventing a disorder or sign or symptom thereof, and/or may be therapeutic in terms of amelioration of the symptoms of the disease or infection, or a partial or complete cure for a disorder and/or adverse effect attributable to the disorder.

As used herein, the given viscosity features are provided through measuring by methods known to one of skill in the art, including the use of various types of viscometers and rheometers.

It is an object of the present invention an artificial diet composition for use in the treatment and/or prevention of cancer comprising, (based on the total weight of the dry ingredients composition):
- from 4 to 40% of a mixture of amino acids,
- from 0 - 25% lipids,
- from 40 - 95% carbohydrates,
- from 1 to 5% of a mixture of vitamins and minerals, and
- from 0 to 1% choline,
characterized in that Leucine is present in the composition as part of the mixture of amino acids in an amount of from 0,5 -6% by weight with respect to the total weight of the dry ingredients composition and Methionine is present in the composition as part of the mixture of amino acids in an amount of from 0,1 - 0,6% by weight with respect to the total weight of the dry ingredients composition.

It is a further object of the present invention an artificial diet composition for use according to any previous paragraph, characterized in that the mixture of amino acids is a mixture of essential and non-essential amino acids, selected from the group consisting of: leucine, isoleucine, valine, methionine, lysine, phenylalanine, tryptophan, threonine, histidine, asparagine, alanine, arginine, aspartic acid, cysteine/cystine, glutamic acid, glutamine, proline, glycine, tyrosine, serine and mixtures thereof.

It is a further object of the present invention an artificial diet composition for use according to any previous paragraph, characterized in that the amino acids are in the free form, salt form, ester form and/or in the form of a peptide, polypeptide or protein.

It is a further object of the present invention an artificial diet composition for use according to any previous paragraph, characterized in that the amino acids present in the composition are a combination of amino acids in the free form and as a protein.

It is a further object of the present invention an artificial diet composition for use according to the previous paragraph, characterized in that the protein is casein.

It is a further object of the present invention an artificial diet composition for use according to any previous paragraph, characterized in that the lipid ingredient is present in an amount of from 0 - 14% by weight with respect to the total weight of the dry composition.

It is a further object of the present invention an artificial diet composition for use according to the previous paragraph, characterized in that the lipid ingredient is selected from any edible vegetable or animal oils, selected from: rapeseed oil, sunflower oil, corn oil, soybean oil, linseed oil, rice oil, safflower oil, olive oil, coconut oil, cottonseed oil, fish oil and mixtures thereof.

It is a further object of the present invention an artificial diet composition for use according to the previous paragraphs, characterized in that the one or more lipid ingredient is selected from the group consisting of: olive oil, coconut oil, salmon oil, corn oil, soybean oil, canola oil, rapeseed oil, sunflower oil, linseed oil, rice oil, safflower oil, cottonseed oil, palm oil, castor seed oil, peanut oil, wheat oil, pumpkin seed oil, poppy seed oil, hemp oil, pomegranate seed oil, cod oil, herring oil, whale oil, seal oil, margarine, butter, lard, tallow, and mixtures thereof..

It is a further object of the present invention an artificial diet composition for use according to any previous paragraph, characterized in that the carbohydrates can be selected from the group consisting of: saccharose, cellulose, starch, and mixtures thereof.

It is a further object of the present invention an artificial diet composition for use according to any previous paragraph, characterized in that it is in a form suitable for oral administration.

It is a further object of the present invention an artificial diet composition for use according to the previous paragraph, characterized in that it is in a solid, semisolid or liquid form and is selected from: dry powder, shake, liquid concentrates, drink ready to drink, chilled or shelf stable beverage, soup, paste, puree, nutritional bar.

It is a further object of the present invention an artificial diet composition for use according to any previous paragraph, characterized in that treatment of cancer comprises: renal cancer, lung cancer, colon cancer, breast cancer, melanoma, ovarian cancer, prostate cancer, pancreatic cancer, liver cancer, endometrial cancer, cervical cancer, bladder cancer, esophageal cancer, gastric cancer, head and neck cancers, leukemia, lymphomas, non-melanoma skin cancers, sarcomas, central nervous system cancers, testicular cancer, thyroid cancer and cancer of unknown primary site.

It is a further object of the present invention an artificial diet composition for use according to any previous paragraph, characterized in that treatment comprises treatment cycles of two to twelve weeks with a four to six daily doses.

It is a further object of the present invention an artificial diet composition for use according to any previous paragraph, characterized in that it further comprises water or a water based carrier.

It is a further object of the present invention a pharmaceutical composition comprising the artificial diet composition of any previous paragraph together with a pharmaceutical acceptable carrier or vehicle, for use in the treatment of cancer.

It is a further object of the present invention a pharmaceutical composition as defined in the previous paragraph for use in the treatment of cancer which comprises: renal cancer, lung cancer, colon cancer, breast cancer, melanoma, ovarian cancer, prostate cancer, pancreatic cancer, liver cancer, endometrial cancer, cervical cancer, bladder cancer, esophageal cancer, gastric cancer, head and neck cancers, leukemia, lymphomas, non-melanoma skin cancers, sarcomas, central nervous system cancers, testicular cancer, thyroid cancer and cancer of unknown primary site.

It is a further object of the present invention a pharmaceutical composition as defined in the previous paragraphs, characterized in that treatment comprises co-administration of the pharmaceutical composition together with any type of drug therapy, including cytotoxic chemotherapy drugs such as alkylating agents (e.g., cisplatin, carboplatin, oxaliplatin, cyclophosphamide, temozolomide, hydroxyurea, etc), antimetabolites (e.g., fluorouracil, capecitabine, gemcitabine, methotrexate, cytarabine etc), mitotic inhibitors (e.g., paclitaxel, docetaxel, cabacitaxel, vincristine, vinblastine, vinorelbine, vindesine, etc), topoisomerase inhibitors (e.g., etoposide, teniposide, irinotecan, topotecan, doxorubicin, epirrubicin, etc), hormonal therapy (e.g., antiestrogens such as tamoxifen, aromatase inhibitors such as anastrozole, LHRH agonists such as goserelin, antiandrogens such as abiraterone and flutamide, corticosteroids such as dexamethasone and prednisone, etc), immunotherapies (e.g., anti-PD1 such as nivolumab, anti-PDL1 such as avelumab, anti-CTLA4 such as ipilimumab, cytokines such as interleukin-2 and interferon, etc), targeted therapies (e.g., anti-VEGF agents such as bevacizumab, anti-VEGFR such as sunitinib and sorafenib, anti-EGFR such as cetuximab or erlotinib, anti-HER2 such as trastuzumab, anti-PARP such as olaparib, anti-BRAF such as vemurafenib, etc), and any other anticancer drug, as well as mixtures thereof.

It is a further object of the present invention a pharmaceutical composition as defined in the previous paragraph, characterized in that such co-administration comprises sequential, concomitant or simultaneous administration of active ingredients.

It is a further object of the present invention a pharmaceutical composition as defined in the previous paragraphs, characterized in that treatment comprises administering to a subject in need thereof of an effective amount of the pharmaceutical composition during surgery and/or radiotherapy treatment.

### DESCRIPTION OF THE FIGURES

Figure 1: Anticancer activity of diet P2, Sunitinib and Anti-PD1 in mice with renal cancer (intraperitoneal model)
Figure 2: Anticancer activity of diet P10 and capecitabine in mice with colon cancer (intraperitoneal model)
Figure 3: Anticancer activity of diet P6 and capecitabine in mice with colon cancer (intravenous model)
Figure 4: Anticancer activity of diet P6, diet P8, cisplatin and capecitabine in mice with triple negative breast cancer (intravenous model)
Figure 5: Cell viability after treatment with diets M0, M1, M2, M3, 5-FU, Cisplatin, Doxorubicin and Paclitaxel

### DETAILED DESCRIPTION OF THE INVENTION

All cancer cells acquire DNA alterations and progress under a relatively constant metabolic environment. Under this normal environment, cancer cells can proliferate uncontrollably, can evade the immune system, and can generally resist most types of systemic therapies. The aim of the present invention is to change the normal metabolic environment of cancer cells by using artificial diets in which the levels and ratios of specific amino acids and lipids are manipulated. Normal cells will use their normal DNA to adapt to the new environment and will resist therapy. Because of their DNA alterations, cancer cells may not be able to fully adapt to the new metabolic environment and may therefore die.

Restricting amino acids can lead to cancer cell death. Briefly, cell survival requires protein synthesis. Proteins are continuously degraded and replaced with new ones to ensure a constant supply of functional proteins _{[23,24]}. Protein synthesis in humans requires adequate levels of the 20 canonical amino acids (AAs). An inadequate supply of just one of them for long enough will jeopardize protein synthesis and will result in cell death. Many proteinogenic AAs are also necessary for other cellular processes. All cancer cells, including CSCs, non-dividing cancer cells, or any type of resistant cancer cell, will die if they do not obtain adequate levels of any proteinogenic AA.

AA restriction can result in selective killing of cancer cells. Human cells cannot synthesize nine of the 20 proteinogenic AAs; these nine AAs are referred to as essential AAs (EAAs) and need to be taken from the diet. The rest, called non-essential AAs (NEAAs), can be synthesized from glucose and from some essential and non-essential AAs. The biosynthesis of NEAAs requires a variety of enzymes that catalyze several reactions and pathways. Some genes encoding these enzymes may not be functional in cancer cells; they may be mutated, silenced or located in lost chromosomes. However, since dietary proteins provide each of the 20 AAs required for protein synthesis, these DNA alterations would not jeopardize the survival of cancer cells. This could change with an artificial diet in which the levels of particular NEAAs are temporarily restricted. Cancer cells with defects in the synthesis of a specific AA would not survive restriction of this AA, while normal cells would. Amino acid manipulation can also be lethal for cancer cells without mutations in genes involved in the synthesis of NEAAs. Carcinogenesis is an evolution process in which normal cells acquire multiple DNA alterations. However, not all of them provide a survival benefit. Since many DNA alterations are incompatible with cell survival under specific environmental conditions, cells can only acquire those alterations that allow them to survive in the existing environment. It is important to realize that carcinogenesis takes place under environments in which the levels and ratios of the 20 proteinogenic AAs remain relatively constant. The main reason is that virtually all food proteins contain each of the 20 proteinogenic AAs, and a standard diet usually provides AAs at relatively constant ratios. However, the present invention proposes altering the environment under which cancer cells have evolved with artificial diets in which the levels of particular AAs are manipulated. This new environment may cause their death, because the DNA alterations that provide a survival benefit under specific environmental conditions may be lethal under other conditions. Scott et al. observed that over 90% of human cancer cells from a wide range of tumors and established cell lines died in vitro following arginine deprivation, while normal cells survived _{[25]}. It is unlikely that all the susceptible cancer cells had mutations in genes involved in the synthesis of the NEAA arginine. Probably, arginine deprivation forced cells to activate a variety of genetic adaptation programs, which were functional in normal cells but not in cancer cells. The accumulation of DNA alterations in cancer cells during carcinogenesis probably inactivated the genetic programs required to adapt to and survive in the new environment created when arginine was deprived.

The authors of the present invention have surprisingly found that in order for an amino acid manipulated diet to be successful, in terms of increase of survival rate of the patient taking such diet when compared within a patient not taking such dietary composition, it is essential to control the intake of specific amino acids and moreover control the amounts of these specific amino acids to be taken, such that the daily intake of a specific combination of amino acids is changed to specific amounts. Contrary to already known amino acid restricted diets of the state of the art, where a group of amino acids is deprived to a certain level or alternatively, completely suppressed, the authors of the present invention have found that anti-tumor activity of an amino acid manipulated diet depends on the interaction of controlled amounts of a group of specific amino acids.

In addition, the activity of the amino-acid-manipulated diet can also depend on the levels of other dietary constituents such as lipids. Lipids participate in multiple cellular processes crucial for tumor development and disease progression. For example, they are essential for the synthesis of the cellular membranes of the new cancer cells, they provide substrates for energy production, they are the starting point for the biosynthesis of cellular mediators highly involved in cancer such as prostaglandin E2 (PGE2). PGE2 promotes angiogenesis, activates the division of cancer stem cells, blocks the type 1 interferon-dependent innate immune response, induces reprograming of macrophages to the M2 subtype, and promotes cancer cachexia _{[26,27]}.

Malnutrition is the most common secondary diagnosis in cancer patients. Even patients who are eating can become malnourished because of specific biochemical and metabolic changes associated with cancer. These metabolic changes impair nutritional status and contribute to cancer-related malnutrition, anorexia, and cachexia. At least 50% of cancer patients are cachetic.za Recent reviews indicate cachexia is even more widespread among patients with advanced cancer.₂₉

Cachexia is derived from the Greek word meaning "bad condition," and is characterized by anorexia (loss of appetite), weight loss, muscle wasting, and chronic nausea. Other noted effects are changes in body composition, alterations in carbohydrate, protein, and lipid metabolism, and depression. Cancer-related metabolic changes lead to preferential depletion of lean body mass as a source of calories. In this way cachexia differs from simple starvation, where the body will metabolize fat stores and protect lean body mass. Anorexia, the loss of appetite and food intake, is noted in 50% of newly diagnosed cancer patients. Early satiety, taste and smell alterations, food aversions, nausea, and vomiting are contributory factors to anorexia.

The anorexia-cachexia syndrome is a major cause of morbidity and mortality in cancer patients. The anorexia/cachexia syndrome, characterized by progressive nutritional changes, weakness, and wasting, is often debilitating and potentially life-threatening over a lengthy period. Therefore, a successful amino-acid-manipulated diet for the treatment of cancer should not only interfere with the proliferation of cancer cells, but should also create a metabolic environment to avoid or improve the anorexia/cachexia syndrome.

The present invention is therefore directed to an artificial diet composition as defined in the claims for use in the treatment of cancer which is characterized by comprising specific amino acids in controlled amounts, present in the composition either in the free, salt, ester form and/or provided through an amino acid source, such as a protein, together with carbohydrates and lipids plus additional ingredients such as mixtures of vitamins and minerals, and optionally a dietary carrier and/or a pharmaceutical carrier.

The artificial diet composition of the present invention comprises from about 4 to 40% by weight of amino acids calculated with respect to the total weight of the dry composition, wherein the content of essential amino acids present in the composition is from 2 to 25% by weight.

Essential amino acids which are present in the composition comprise: leucine, isoleucine, valine, methionine, lysine, phenylalanine, tryptophan, threonine, histidine and mixtures thereof. In a particular embodiment of the present invention, the essential amino acids: leucine and methionine are present in the composition in controlled amounts. Non-essential amino acids which can be present in the composition are selected from the group consisting of asparagine, alanine, arginine, aspartic acid, cysteine/cystine, glutamic acid, glutamine, proline, glycine, tyrosine, serine and mixtures thereof.

Other amino acids, such as, betaine, taurine etc. can also form part of the composition, as well as amino acid metabolites and/or precursors thereof.

The compositions according to the present invention comprise Leucine in an amount of from 0.5 - 6 % by weight and Methionine in an amount of from 0,1 - 0,6% by weight with respect to the total weight of the dry composition.

In a preferred embodiment of the invention, in addition to Leucine in an amount of from 0.5 - 6 % by weight and Methionine in an amount of from 0,1 - 0,6% by weight, Glutamine is present in an amount of 0 - 10% and Cysteine/Cystine is present in an amount of 0 - 0.5% by weight with respect to the total weight of the dry composition.

Preferred amounts of Leucine in the composition are: 0,5%, 2,5%; 5%, 6% and 10% by weight with respect to the total weight of the dry composition.

Preferred amounts of Methionine in the composition are: 0,17%; 0,5% and 0,6% by weight with respect to the total weight of the dry composition.

Preferred amounts of Glutamine in the composition are: 1,3%, 5% and 6% by weight with respect to the total weight of the dry composition.

Preferred amounts of Cysteine/Cystine in the composition are: 0%, 0,2% and 0,5% by weight with respect to the total weight of the dry composition.

Suitably, the amino acids present in the dietary composition of the present invention may be amino acids in free form, salts, ester and/or in the form of an amino acid source, such as a polypeptide, a peptide, a protein, an amino acid metabolite and/or precursor. Proteins present in the composition as a source of amino acids can be selected from the group consisting of: casein, and any other protein that provides the levels of amino acids described in this invention, such as collagen, albumin, globulin, ovalbumin, zein, fibroin, keratin, gelatin, gluten, whey protein, egg protein, pea protein, hemp protein, soy protein, and plant and animal protein isolates. In a preferred embodiment, the composition of the present invention comprises casein as a source of amino acids, alone or in combination with additional amino acids in free form, salts or esters.

Such amino acids in free form, salts or ester are commercially available from, for example, Applichem, Acros Organics, ProFoods, BulkSupplements.com, Blackburn Distributions, Myprotein, etc.

The composition of the present invention comprises in addition to amino acids as discussed in the preceding paragraphs, one or more lipid ingredients in an amount of from 0 to 25% by weight with respect to the total weight of the dry composition. In a preferred embodiment of the present invention, the one or more lipid ingredients are present in an amount of from 0 - 14% by weight with respect to the total weight of the dry composition. Suitable lipid ingredients for the purpose of the present invention's compositions may be selected from any edible vegetable or animal oil, such as, rapeseed oil, sunflower oil, corn oil, soybean oil, linseed oil, rice oil, safflower oil, olive oil, coconut oil, cottonseed oil, fish oil and the like. In a preferred embodiment of the present invention, the lipid ingredient is selected from the group consisting of: olive oil, coconut oil, salmon oil, and mixtures thereof.

Such lipid ingredients are commercially available, for example, from local markets.

The composition of the present invention comprises in addition to amino acids and lipids as defined in any of previous paragraphs, one or more carbohydrates in an amount of from 40 to 95% by weight with respect to the total weight of the dry composition. The carbohydrate portion of the composition can be supplied by any suitable carbohydrate source, for example, starches, dextrins, glycogen, glucose, fructose, sugars, monosaccharides, disaccharides, oligosaccharides, polysaccharides, and mixtures thereof. Preferred carbohydrates in accordance with the present invention can be selected from the group consisting of: saccharose, cellulose and starch and mixtures thereof.

Such carbohydrates are commercially available from, for example, local markets, ProFoods, BulkSupplements.com, Blackburn Distributions, Myprotein, etc.

The composition of the present invention comprises in addition to amino acids, lipids and carbohydrates as defined in any of previous paragraphs, a mixture of vitamins and minerals in an amount of from 1 to 5% by weight with respect to the total weight of the dry composition.

Suitable Mixtures of vitamins and minerals to be used in the compositions of the present invention can be selected from the group consisting of: calcium carbonate, monopotassium phosphate, potassium citrate, sodium chloride, potassium sulfate, magnesium oxide, ferric citrate, zinc carbonate, manganese carbonate, copper carbonate, potassium iodate, sodium selenate, ammonium paramolybdate-tetrahydrate, sodium metasilicate-nonahydrate, chromium potassium sulfate-dodecahydrate, lithium chloride, boric acid, sodium fluoride, nickel carbonate hydroxide, ammonium meta-vanadate, Thiamine Hydrocloride, Riboflavin, Pyridoxine Hydrochloride, Nicotinic acid, D-Calcium Pantothenate, Folic Acid, D-Biotin, Cyanocobalamin (Vitamin B12), Retinyl Palmitate (Vitamin A) Pre-mix (250,000IU/g), DL-a-Tocopherol Acetate (250IU/g), Cholecalciferol (Vitamin D3, 400,000 IU/g), Menaquinone (Vitamin K2) and mixtures thereof.

The composition comprises additionally choline in free form, salts, ester, such as choline chloride or choline bitartrate, in an amount of from 0 to 1%, preferably 0,25% by weight with respect to the total weight of the dry composition.

Such vitamin and mineral mixtures are commercially available, for example, from Fisher Bioreagents, MP biomedical, etc.

The dietary compositions of the present invention may additionally contain optional ingredients selected from the group consisting of: stabilizers, preservatives, emulsifiers and flavorings, as commonly known from "Formulation Engineering of Foods", Wiley-Blackwell, August 2013 and "Handbook of Food Chemistry" Springer, 2015.

The dietary product of the present invention is suitable for oral administration and therefore will present any form from liquid to solid with corresponding consistencies and viscosities to meet swallowing needs, such as clear liquid, soft, or full solid diet. Suitable forms comprise: powder, shake, liquid concentrates, drink ready to drink, chilled or shelf stable beverage, soup, paste, puree, nutritional bar, etc.

In a further embodiment of the present invention, the composition of the present invention may optionally comprise in addition to any of the above referred ingredients, water or water based carriers to form the artificial diet of the present invention.

Water or any water based carriers can be mixed with the ingredients of the composition listed in preceding paragraphs, as required, to form the artificial diet composition of the present invention with the desired consistency. The artificial diet composition of the present invention is present in the form of different consistencies and viscosities depending on the amount of water or water based carrier added in the composition so as to address health conditions of the subject which compromise oral intake of the composition, such as for example, dysphagia. The different consistencies and viscosities range from fluid to semi-solid and solid forms, with viscosities ranging from 0,001 to 0,05 Pa.s for the thin liquids, 0,051 to 0,35 Pa.s range for nectar-like liquids, 0,351 to 1,75 Pa.s for the honey-like liquids and not less than 1,751 Pa.s for spoon-thick liquids.

Alternatively, the composition of the present invention does not contain water or water based carriers, so that the composition is present in a solid to semi-solid dry form. The solid or semi-solid dry composition is ready for consumption. Optionally, such composition can be mixed with water before consumption so as to reach the desired volume of final artificial diet in liquid form.

It is also an additional embodiment of the present invention the provision of a pharmaceutical composition comprising the dietary composition of the present invention as defined in any of the preceding paragraphs and in the claims together with pharmaceutically acceptable carriers, excipients or diluents. These pharmaceutical compositions are formulated to be administered to a patient orally, enterally, rectally, vaginally, parenterally, intrapulmonary, sublingually, pulmonary and or intranasal. The present invention is therefore preferably directed to a pharmaceutical formulation, wherein the formulation is in the form of a solid or liquid; and wherein the formulation is in the form of a tablet, a capsule, a gel tab, a lozenge, an orally dissolved strip, syrup, an oral suspension, an emulsion, a granule, a sprinkle and a pellet. The formulation of any solid or liquid pharmaceutical dosage form comprising the dietary composition of the present invention is well known and customary practice for a person skilled in the art. The pharmaceutical carriers, excipients and diluents for manufacturing said pharmaceutical compositions are well known for a skilled person in the art, see Remington, The Science and Practice of Pharmacy, 21st Edition; Lippincott Williams& Wilkins *and* Handbook of Pharmaceutical Excipients, 5th Edition, Rowe *et al.*

The artificial diet composition of the present invention provides all of the daily nutritional requirements of a subject and is to be taken as a replacement of meal. In order to fulfill the daily nutritional requirements of the subject, the artificial diet composition of the present invention can be taken once, twice, or in multiple doses as required to complete the necessary daily caloric intake. In a preferred embodiment the artificial diet is taken in 4 to 6 daily doses.

As will be shown in the experimental part of the description, daily intake of the artificial diet composition of the present invention provides an enhanced anti-cancer effect when compared to therapeutic treatment with conventionally drugs, such as, sunitinib, anti-PD1, capecitabine or cisplatin. Thus, the artificial diet composition of the invention is suitable for use as the sole active agent in the treatment of cancer. Alternatively, the artificial diet composition or pharmaceutical composition comprising the artificial diet of the present invention is suitable for use in the treatment of cancer in a co-administration regime with other anti-cancer drugs, such treatment regime comprising sequential, concomitant or simultaneous administration of active ingredients. Anti-cancer drugs contemplated to be used in combination with the artificial diet composition or pharmaceutical composition comprising the artificial diet of the present invention are selected from the group comprising: cytotoxic chemotherapy drugs such as alkylating agents (e.g., cisplatin, carboplatin, oxaliplatin, cyclophosphamide, temozolomide, hydroxyurea, etc), antimetabolites (e.g., fluorouracil, capecitabine, gemcitabine, methotrexate, cytarabine etc), mitotic inhibitors (e.g., paclitaxel, docetaxel, cabacitaxel, vincristine, vinblastine, vinorelbine, vindesine, etc), topoisomerase inhibitors (e.g., etoposide, teniposide, irinotecan, topotecan, doxorubicin, epirrubicin, etc), hormonal therapy (e.g., antiestrogens such as tamoxifen, aromatase inhibitors such as anastrozole, LHRH agonists such as goserelin, antiandrogens such as abiraterone and flutamide, corticosteroids such as dexamethasone and prednisone, etc), immunotherapies (e.g., anti-PD1 such as nivolumab, anti-PDL1 such as avelumab, anti-CTLA4 such as ipilimumab, cytokines such as interleukin-2 and interferon, etc), targeted therapies (e.g., anti-VEGF agents such as bevacizumab, anti-VEGFR such as sunitinib and sorafenib, anti-EGFR such as cetuximab or erlotinib, anti-HER2 such as trastuzumab, anti-PARP such as olaparib, anti-BRAF such as vemurafenib etc), and any other anticancer drug as well as mixtures thereof.

The dietary compositions of the present invention may be taken for approximately two to twelve weeks, one or several times depending on the evolution of the disease. After a 2-12 week treatment with the metabolic diet, the patient returns to a normal diet for a predetermined period, such as one, two, three, etc. weeks. The daily dosages contemplated for providing the desired therapeutic effect are those that provide the required caloric needs for the person. Note that the term calorie is commonly used as shorthand for kilocalorie (Kcal), and that the caloric needs of a person depend on the age, gender, height, weight and physical activity. The calories (Kcal) provided by a metabolic diet can be estimated by considering that 1 g of carbohydrates provides 4.1 calorie (Kcal), 1 g of protein (or amino acids) provides 4.1 calorie, 1 g of fat (lipids) provides 8.8 calorie, and 1 g of fiber provides 1.9 calorie. For example, the caloric needs for a person with the following characteristics (man, 50 years, 175 cm, 75 Kg, little or no exercise) are approximately 1900 (https://www.calculator.net/bmr-calculator.html). If this person is treated with diet P9, he would have to take approximately 500 g of the dry composition each day (500 g of this diet provides around 1900 calorie). This total daily amount can be divided into 4-6 takes per day; for example, 100g at 8:00 h, 100g at 12:00, 100g at 16:00, 100g at 20:00, and 100g at 24:00. Note that during treatment, the patient should only take these amounts of the dry compositions (which can be prepared with different amounts of water) and should only drink water.

### Cancer treatment:

The present invention provides an artificial diet composition and/or a pharmaceutical composition for use in a method of treating cancer.

The present invention also provides a method of treating cancer in a subject in need thereof, comprising administering to the subject in need thereof an effective amount of the dietary composition and/or the pharmaceutical composition of the present invention. Treating cancer according to the present invention comprises any type of cancer, including renal cancer, lung cancer, colon cancer, breast cancer, melanoma, ovarian cancer, prostate cancer, pancreatic cancer, liver cancer, endometrial cancer, cervical cancer, bladder cancer, esophageal cancer, gastric cancer, head and neck cancers, leukemia, lymphomas, non-melanoma skin cancers, sarcomas, central nervous system cancers, testicular cancer, thyroid cancer, cancer of unknown primary site, etc.

### Method of preparing the compositions of the invention

Diets were prepared by mixing all the solid ingredients shown in the tables at ambient temperature according to well-known methods until they formed a well-blended dry powder in a semi-solid to a solid state depending on the amount of lipid ingredients used in each case.

Mineral Mix (Harlan Laboratories, AIN-93M-MX) constituted 3.5% of the dry diet; 100g of dry diet contained 1.25% calcium carbonate, 0.875% monopotassium phosphate, 0.098% potassium citrate, 0.259% sodium chloride, 0.163% potassium sulfate, 0.085% magnesium oxide, 0.021% ferric citrate, 0.0058% zinc carbonate, 0.0022% manganese carbonate, 0.0011% copper carbonate, 0.000035% potassium iodate, 0.000035% sodium selenate, 0.000028% ammonium paramolybdate-tetrahydrate, 0.0051% sodium metasilicate-nonahydrate, 0.00095% chromium potassium sulfate-dodecahydrate, 0.0000595% lithium chloride, 0.000284% boric acid, 0.00022% sodium fluoride, 0.00011% nickel carbonate hydroxide, 0.000021% ammonium meta-vanadate and 0.73% sucrose.

Vitamin mix (AIN Vitamin Mixture 76) constituted 1% of the dry diet; 100g of dry diet contained (mg) Thiamine Hydrocloride (0,6), Riboflavin (0,6), Pyridoxine Hydrochloride (0,7), Nicotinic acid (3), D-Calcium Pantothenate (1,6), Folic Acid (0,2), D-Biotin (0,02), Cyanocobalamin (Vitamin B12) (0,001), Retinyl Palmitate (Vitamin A) Pre-mix (250,000IU/g) (1,6), DL-a-Tocopherol Acetate (250IU/g) (20), Cholecalciferol (Vitamin D3, 400,000 IU/g) (0,25), Menaquinone (Vitamin K2) (0,005), sucrose (972,9).

Casein was obtained from Acros Organics. Amino acids were obtained from different sources, including Applichem, Acros Organics and Myprotein. Choline (bitartrate) was obtained from Acros Organics, Olive oil, coconut oil and sucrose were obtained from local markets. Corn starch and cellulose were obtained from Farmusal (local pharmacy).

Preferred compositions (Table 1 and 2) were obtained following the method described in preceding paragraphs:

**Table 1: Preferred compositions. The typical amount (g) of amino acids in 100 g and 6 g (shown in brackets) of the casein used in the experiments is: Glutamine + Glutamate: 21,7 (1,302), Leucine: 9 (0,54), Methionine: 2,9 (0,174), Phenylalanine: 4,8 (0,288), Histidine: 2,6 (0,156), Lysine: 7,5 (0,45), Threonine: 4,1 (0,246), Isoleucine: 4,3 (0,258), Valine: 5,3 (0,318), Tryptophan: 1,2 (0,072), Cysteine/cystine: 0,7 (0,042), Arginine: 3,4 (0,204), Glycine: 1,7 (0,102), Serine: 5,7 (0,342), Tyrosine: 5,2 (0,312), Alanine: 2,9 (0,174), Aspartate + Asparagine: 6,9 (0,414), Proline: 10,1(0,606).**

| **Diet** | **P1** | **P2** | **P3** | **P4** | **P5** | **P6** | **P7** | **P8** | **P9** | **P10** | **P11** | **P12** |
|---|---|---|---|---|---|---|---|---|---|---|---|---|
| **Casein** | | | | | | | 6 | 6 | 6 | 6 | 6 | 6 |
| **Glutamine** | 6 | 6 | 6 | 6 | 6 | 6 | 5 | 5 | | 5 | | |
| **Leucine** | 6 | 6 | 6 | 6 | 6 | 6 | | 5 | 2,5 | 2,5 | | |
| **Methionine** | 0,6 | 0,6 | 0,6 | 0,6 | 0,6 | 0,17 | | | | | | |
| **Phenylalanine** | 2,16 | 2,16 | 2,16 | 2,16 | 2,16 | 0,6 | | | | | | |
| **Histidine** | 0,85 | 0,85 | 0,85 | 0,85 | 0,85 | 0,24 | | | | | | |
| **Lysine** | 2,64 | 2,64 | 2,64 | 2,64 | 2,64 | 0,73 | | | | | | |
| **Threonine** | 1,8 | 1,8 | 1,8 | 1,8 | 1,8 | 0,5 | | | | | | |
| **Isoleucine** | 1,07 | 1,07 | 1,07 | 1,07 | 1,07 | 0,3 | | | | | | |
| **Valine** | 2,64 | 2,64 | 2,64 | 2,64 | 2,64 | 0,73 | | | | | | |
| **Tryptophan** | 0,24 | 0,24 | 0,24 | 0,24 | 0,24 | 0,07 | | | | | | |
| **Cystine** | | | | | 0,2 | | | | | | | |
| **Arginine** | | 1,5 | 1,5 | | | | | | | | | |
| **Glycine** | | 1 | 1 | | | | | | | | | |
| **Serine** | | | | | | | | | | | | |
| **Tyrosine** | | | 1 | | | | | | | | | |
| **Alanine** | | 1 | 1 | | | | | | | | | |
| **Aspartate** | | 2 | 2 | | | | | | | | | |
| **Proline** | | | | | | | | | | | | |
| **Asparagine** | | | | | | | | | | | | |
| **Glutamate** | | | | | | | | | | | | |
| **Olive oil** | 14 | 14 | 14 | 1 | 1 | | | | | | | |
| **Coconut oil** | | | | | | 1 | 1 | | | | | |
| **Salmon oil** | | | | | | | | 1 | 1 | 1 | 1 | |
| **Choline** | 0,25 | 0,25 | 0,25 | 0,25 | 0,25 | 0,25 | 0,25 | 0,25 | 0,25 | 0,25 | 0,25 | 0,25 |
| **Vitamin Mix** | 1 | 1 | 1 | 1 | 1 | 1 | 1 | 1 | 1 | 1 | 1 | 1 |
| **Mineral Mix** | 3,5 | 3,5 | 3,5 | 3,5 | 3,5 | 3,5 | 3,5 | 3,5 | 3,5 | 3,5 | 3,5 | 3,5 |
| **Sucrose** | 15 | 15 | 15 | 15 | 15 | 15 | 15 | 15 | 15 | 15 | 15 | 15 |
| **Celulose** | 5 | 5 | 5 | 5 | 5 | 5 | 5 | 5 | 5 | 5 | 5 | 5 |
| **Corn starch** | 37,25 | 31,75 | 30,75 | 50,25 | 50,05 | 58,91 | 63,25 | 58,25 | 65,75 | 60,75 | 68,25 | 69,25 |
| | | | | | | | | | | | | |
| **Total (g or %)** | 100 | 100 | 100 | 100 | 100 | 100 | 100 | 100 | 100 | 100 | 100 | 100 |

**Table 2: Preferred compositions**

| **Diet** | **P13** | **P14** | **P15** | **P16** | **P17** | **P18** | **P19** | **P20** | **P21** | **P22** | **P23** | **P24** |
|---|---|---|---|---|---|---|---|---|---|---|---|---|
| **Casein** | | | | | | | | | | 6 | 6 | 6 |
| **Glutamine** | 6 | 6 | 6 | 6 | 6 | 6 | 6 | 6 | 6 | | | |
| **Leucine** | 6 | 6 | 6 | 6 | 6 | 6 | 10 | 6 | 6 | | | |
| **Methionine** | 0,6 | 0,6 | 0,6 | 0,6 | 0,6 | 0,6 | 0,6 | 0,6 | 0,6 | | 0,5 | 0,5 |
| **Phenylalanine** | 2,16 | 2,16 | 2,16 | 2,16 | 2,16 | 2,16 | 2,16 | 2,16 | 2,16 | | | |
| **Histidine** | 0,85 | 0,85 | 0,85 | 0,85 | 0,85 | 0,85 | 0,85 | 0,85 | 0,85 | | | |
| **Lysine** | 2,64 | 2,64 | 2,64 | 2,64 | 2,64 | 2,64 | 2,64 | 2,64 | 2,64 | | | |
| **Threonine** | 1,8 | 1,8 | 1,8 | 1,8 | 1,8 | 1,8 | 1,8 | 1,8 | 1,8 | | | |
| **Isoleucine** | 1,07 | 1,07 | 1,07 | 1,07 | 1,07 | 1,07 | 1,07 | 1,07 | 1,07 | | | |
| **Valine** | 2,64 | 2,64 | 2,64 | 2,64 | 2,64 | 2,64 | 2,64 | 2,64 | 2,64 | | | |
| **Tryptophan** | 0,24 | 0,24 | 0,24 | 0,24 | 0,24 | 0,24 | 0,24 | 0,24 | 0,24 | | | |
| **Cystine** | | | | 1 | | | | | | 0,5 | | 0,5 |
| **Arginine** | 1,5 | 1,5 | 1,5 | 1,5 | 1,5 | 1,5 | 1,5 | 1,5 | 1,5 | | | |
| **Glycine** | 1 | | 1 | 1 | 1 | 1 | 1 | 1 | 1 | | | |
| **Serine** | 1,5 | | | | | | | | | | | |
| **Tyrosine** | | | | | | | | | | | | |
| **Alanine** | 1 | 1 | 1 | 1 | 1 | 1 | 1 | 1 | 1 | | | |
| **Aspartate** | 2 | 2 | 2 | 2 | 2 | 2 | 2 | 2 | 2 | | | |
| **Proline** | | | | | 1,5 | | | | | | | |
| **Asparagine** | | | 1,5 | | | | | | | | | |
| **Glutamate** | | | | | | 2 | | | | | | |
| **Olive oil** | 14 | 14 | 14 | 14 | 14 | 14 | 14 | 5 | 25 | | | |
| **Coconut oil** | | | | | | | | | | | | |
| **Salmon oil** | | | | | | | | | | 1 | 1 | 1 |
| **Choline** | 0,25 | 0,25 | 0,25 | 0,25 | 0,25 | 0,25 | 0,25 | 0,25 | 0,25 | 0,25 | 0,25 | 0,25 |
| **Vitamin Mix** | 1 | 1 | 1 | 1 | 1 | 1 | 1 | 1 | 1 | 1 | 1 | 1 |
| **Mineral Mix** | 3,5 | 3,5 | 3,5 | 3,5 | 3,5 | 3,5 | 3,5 | 3,5 | 3,5 | 3,5 | 3,5 | 3,5 |
| **Sucrose** | 15 | 15 | 15 | 15 | 15 | 15 | 15 | 15 | 15 | 15 | 15 | 15 |
| **Celulose** | 5 | 5 | 5 | 5 | 5 | 5 | 5 | 5 | 5 | 5 | 5 | 5 |
| **Corn starch** | 30,25 | 32,75 | 30,25 | 30,75 | 30,25 | 29,75 | 27,75 | 40,75 | 20,75 | 67,75 | 67,75 | 67,25 |
| | | | | | | | | | | | | |
| **Total (g or %)** | 100 | 100 | 100 | 100 | 100 | 100 | 100 | 100 | 101 | 100 | 100 | 100 |

Hereinafter, the present invention will be described in more detail with reference to specific examples. However, these examples are only for illustrating the present invention in more detail, the scope of the present invention is therefore not limited by these examples.

### EXAMPLES

### Experimental Conditions

### Cell lines and cell culture conditions

A549 cell line (human non-small cell lung cancer) was purchased from European Collection of Authenticated Cell Cultures (ECACC). 786-O (renal cancer), MDA-MB-231 (triple negative breast cancer), LLc1 (murine lung cancer), Renca (murine renal cancer), 4T1 (murine breast cancer), CT26WT (murine colorectal cancer) and B16-F10 (murine melanoma) were obtained from American Type Culture Collection (ATCC). A64-CLS (submaxillary gland adenoma), AN3Ca (endometrial adenocarcinoma), BT-474 (breast cancer; Luminal B (ER+; PR+; Her-2 +), Calu-1 (squamous lung cancer), HNO97 (tongue cancer), MeWo (melanoma; BRAF WT), NIH:OVCAR-3 (ovarian cancer), Sk-Br-3 (breast cancer; HER-2 +), Sk-OV-3 (ovarian cancer), T24 (bladder cancer), T-47D (breast cancer; Luminal A (ER+; PR+; Her-2 -) and HaCaT cell line (skin normal) were purchased from Cell Lines Service (CLS). UACC-62 (melanoma; BRAF mut) was obtained from National Cancer Institute (Rockville, MD). CAPAN-1 (pancreatic cancer), HepG2 (human hepatocellular carcinoma), HT29 (colorectal cancer) and PC3 (human prostate cancer) were generously provided by Dr. Helleday (Karolinska Institute, Sweden). GAMG (glioblastoma) was kindly provided by Dr. Ayala (University of Seville, Spain). A549, A64-CLS, AN3Ca, B16-F10, BT-474, GAMG, HaCaT, HepG2, HNO97, HT29, LLc1, MDA-MB-231, MeWo, Sk-Br-3, Sk-OV-3 and T24 were cultured in Dulbecco's modified Eagle's medium (DMEM) high glucose medium. 4T1, 786-O, Calu-1, CAPAN-1, CT26WT, NIH:OVCAR-3, PC-3, Renca, T-47D, UACC-62 were grown in RPMI 1640. All media were supplemented with 100 U/mL penicillin, 100 µg/mL streptomycin and 10% fetal bovine serum. All cells were kept at 37°C in a humidified atmosphere containing 5% CO2. Cell culture reagents were purchased from Biowest or Thermo Fisher Scientific.

### Cell viability assay.

Exponentially growing cells were seeded in 96-well plates and were allowed to grow during 24h. The cells were then exposed to the artificial media or to several concentrations of the anticancer drugs for 7 days. Then, the cells were allowed to recover in their corresponding standard media (drug-free) during 3 days. Cell viability was then estimated with the resazurin assay. This assay is a redox-based colorimetric technique based on the capability of viable cells to reduce the blue reagent resazurin into a pink-colored product. The number of live cells is directly proportional to the amount of final product formed. After treatments and the recovery period, medium was removed, and 150 µL of resazurin solution (20 µg/mL in medium) was added to each well for 5-7 h (depending on the cell line). The optical densities of each well were measured at 540 nm and 620 nm on a multi-well plate spectrophotometer reader. Results were expressed as percentage of cell viability in relation to untreated cells grown in their standard media. Data for the artificial media were averaged from at least three independent experiments and were expressed as the means ± standard error of the mean (SEM). Data for the anticancer drugs were averaged from two wells of one experiment (data were consistent with those that authors routinely obtain in their laboratory with these drugs).

### Mice and experimental in vivo conditions

All mice were purchased from Janvier Labs^{®} (France). Male BALB/cAnNRJ mice were used for the renal cancer model (Renca cells, intraperitoneal model). Female BALB/cAnNRJ mice were used for the colon cancer models (CT26WT cells, intraperitoneal and intravenous models), and for the triple negative breast cancer model (4T1 cells, intravenous model). Female C57BL/6J mice were used for the lung cancer model (LLc1 cells, intravenous model) and for the melanoma model (B16-F10 cells, intravenous model). All mice were 12 weeks or older at the beginning of the experiments. Treatments started 8 days after the injection of the cancer cells for the 4T1 breast cancer model and for the Renca renal cancer model (when the number of injected cells was 100.000). Treatments started 7 days after the injection for the Renca cancer model when the number of inoculated cells was 150.000, and for the LLc1 lung cancer model. Treatments started 4 days after the injection in the CT26WT colon cancer models (intraperitoneal and intravenous) and in the B16-F10 melanoma model.

Murine cells (5th-7th passage) were cultured in 75-cm2 flask. When the cells were approximately 60-70% confluent, medium was removed and cells were washed twice with sterile PBS. Then, cells were incubated with trypsin/EDTA solution for 2-3 min at 37 °C to allow cells to have a rounded shape but without detaching. Next, trypsin/EDTA solution was aspirated, cells were resuspended in 5 mL sterile PBS and the cell suspension was pipetted up and down to break up any cell aggregate before adding 10% FBS supplemented medium. Then, a working cell suspension (between 5×10⁵-25×10⁶cells/mL depending on cancer model) was prepared. This suspension was centrifuged (5 min, 250 g) at room temperature. Medium was removed and cells were resuspended in warm 2,5% FBS supplemented medium. The working cell suspension was aliquoted into 2 mL tubes and they were kept at 37°C in a humidified atmosphere containing 5% CO2 until use. Several minutes before the injection, tubes were centrifuged at 300 g 4°C for 3 min, medium was removed and cells were resuspended in sterile PBS. Cells were counted again. Finally, a 1-mL syringe (insulin type with a 29-G × 1/2"needle) was filled with 0.2 mL of the working cell suspension, which was injected in the peritoneal cavity or in the tail vein of the mice. After inoculating the last mice, the cells in the last tube were placed in a flask and monitored during several weeks under the microscope to ensure that all mice were inoculated with viable cells.

One day before the start of the treatments, mice were housed in individual cages to avoid cannibalism. Treatments started four, seven or eight days (depending on the model) after injecting the cancer cells. Most treatments with the artificial diets lasted at least 4 weeks. Treatment with the artificial diets simply consisted of replacing their normal diet with an artificial diet in which the levels of specific AAs and lipids were manipulated. Sunitinib, and capecitabine were administered daily in the diet. Cisplatin and the anti-PD1 antibody were injected intraperitoneally. Animals were monitored daily and body weights were determined periodically (at least three times per week). Mice were euthanized by cervical dislocation when signs of disease progression were apparent; these signs (e.g., excessive gains and losses of body weights, reduced mobility and curiosity, respiratory distress, and/or visible or palpable tumors exceeding 15-20 mm) indicated that survival for additional 48 h was unlikely. Post-mortem examination was carried out to confirm the cause of death and to observe the extent of the disease. Autopsies confirmed the presence of tumors in all euthanized mice.

### Diet Preparation

Diets were prepared by first mixing all the solid ingredients shown in the tables until they formed a well-blended dry powder. After adding the oil (if present in the composition) to the mixture, enough water was added bit by bit to make a soft dough. The dough was left air-dried for about 2 h, pelleted manually (approximately 5 g/pellet), left air-dried for additional 2 h, and stored until use.

Mineral Mix (Harlan Laboratories, AIN-93M-MX) constituted 3.5% of the dry diet; 100g of dry diet contained 1.25% calcium carbonate, 0.875% monopotassium phosphate, 0.098% potassium citrate, 0.259% sodium chloride, 0.163% potassium sulfate, 0.085% magnesium oxide, 0.021% ferric citrate, 0.0058% zinc carbonate, 0.0022% manganese carbonate, 0.0011% copper carbonate, 0.000035% potassium iodate, 0.000035% sodium selenate, 0.000028% ammonium paramolybdate-tetrahydrate, 0.0051% sodium metasilicate-nonahydrate, 0.00095% chromium potassium sulfate-dodecahydrate, 0.0000595% lithium chloride, 0.000284% boric acid, 0.00022% sodium fluoride, 0.00011% nickel carbonate hydroxide, 0.000021% ammonium meta-vanadate and 0.73% sucrose.

Vitamin mix (AIN Vitamin Mixture 76, Fisher Bioreagents) constituted 1% of the dry diet; 100g of dry diet contained (mg) Thiamine Hydrocloride (0,6), Riboflavin (0,6), Pyridoxine Hydrochloride (0,7), Nicotinic acid (3), D-Calcium Pantothenate (1,6), Folic Acid (0,2), D-Biotin (0,02), Cyanocobalamin (Vitamin B12) (0,001), Retinyl Palmitate (Vitamin A) Pre-mix (250,000IU/g) (1,6), DL-a-Tocopherol Acetate (250IU/g) (20), Cholecalciferol (Vitamin D3, 400,000 IU/g) (0,25), Menaquinone (Vitamin K2) (0,005), sucrose (972,9). Casein was obtained from Acros organic (27607; bovine). The typical amount (g) of amino acids in 100 g and 6 g (shown in brackets) of the casein used in the experiments is: Glutamine + glutamate: 21,7 (1,302), Leucine: 9 (0,54), Methionine: 2,9 (0,174), Phenylalanine: 4,8 (0,288), Histidine: 2,6 (0,156), Lysine: 7,5 (0,45), Threonine: 4,1 (0,246), Isoleucine: 4,3 (0,258), Valine: 5,3 (0,318), Tryptophan: 1,2 (0,072), Cysteine/cystine: 0,7 (0,042), Arginine: 3,4 (0,204), Glycine: 1,7 (0,102), Serine: 5,7 (0,342), Tyrosine: 5,2 (0,312), Alanine: 2,9 (0,174), Aspartate + Asparagine: 6,9 (0,414), Proline: 10,1(0,606). Amino acids were obtained from different sources, including Applichem, Acros Organics and Myprotein. Choline (bitartrate) was obtained from Acros Organics, Olive oil, coconut oil and sucrose were obtained from local markets. Salmon oil was obtained from Petspurest. Corn starch and cellulose were obtained from Farmusal (local pharmacy).

### Drugs

Sunitinib malate (462640010, Acros Organics) was mixed in the food. Mice were fed a standard diet supplemented with sunitinib (350 mg/kg diet) for 28 days. A 25-g mouse typically consumed an average of 4,5 g diet per day, which results in a dose of approximately 60 mg/kg/day. The normal diet was powdered and mixed with sunitinib. Then enough water was added to make a soft dough, which was left air-dried for about 2 h, pelleted manually (approximately 5 g/pellet) and stored until use. Capecitabine (500mg/pill, 707278.2, Normon) was also mixed in the food (following the process described for sunitinib). Mice were fed with a standard diet supplemented with capecitabine (2500 mg/kg diet) for 7 days, followed by 7-day drug-free normal food. Mice received two-three cycles depending on their state of health. A 25-g mouse typically consumed an average of 4,5 g diet per day, which results in a dose of approximately of 450 mg/kg/day. Cisplatin (1 mg/mL, 659219.9, Cisplatin Pharmacia, Pfizer) was administered intraperitoneally once a week for 4 weeks. Mice received a 5 mg/kg dose in each dose. Anti-PD-1 (anti-mouse PD-1 (CD279), clone RMP1-14, BE0146, Bioxcell) was administered intraperitoneally every 4 days for a total of 4 doses. In each dose, mice received 250 µg. Anti-PD-1 was diluted in pH 7.0 Buffer (InVivoPure, IP0070, Bioxcell). In the in vitro experiments we also used the following anticancer drugs: Doxorubicin (50 mg powder for solution, 958314.9, Farmiblastina, Pfizer), 5-Fluorouracil (F6627, sigma) and Paclitaxel (66997, TEVA, 6 mg/ml).

### In vivo activity results

Table 3 shows results from one experiment in mice with renal cancer treated with several diets. In most of the diets, one amino acid was added or eliminated with respect to diet P2. Previous amino acid restricted diets (e.g., WO 2017/144877) indicated that the activity of the diets depends on the elimination of one or several amino acids such as serine and glycine. This experiment clearly shows that the elimination of serine is not required for activity; in fact, mice treated with the diet containing serine (P13) live longer than the mice treated with the same diet without serine (P2). Previous data also indicated that the elimination of both serine and glycine is important for activity; however, results shown in Table 3 indicate that elimination of both amino acids (diet P14) is worse than the diet that contains both serine and glycine (P13) or than the diet that contains glycine but not serine (P2). In addition, this experiment also shows that an increase in the levels of lipids can reduce the activity of the composition (diet P21 is worse than diet P2) and that a reduction in the levels of lipids can increase the activity of the diet (Diet P20 is better than diet P2). Therefore, contrary to already known amino acid restricted diets of the state of the art, where a group of amino acids is deprived to a certain level or completely suppressed, these results shows that anti-tumor activity of an amino acid restricted diet depends on the interaction of controlled amounts of a group of specific amino acids, and also depends on their interaction with other dietary constituents such as lipids.

**Table 3: Survival of mice with renal cancer treated with several metabolic diets. Male BALB/cAnNRJ mice with renal cell carcinoma were treated with anti-PD1 antibody (250µg administered intraperitoneally on days 8, 12, 16 and 20), with one of the following diets: P2, P3, P13, P14, P15, P16, P17, P18, P19, P20, P21 (normal diet was replaced by one of these diets during 28 days), or left untreated (control, normal diet). Treatments started 8 days after the intraperitoneal injection of 100,000 Renca cancer cells At least three mice were included in each group Mice were euthanized by cervical dislocation when signs of disease progression were apparent; these signs (e.g., excessive gains and losses of body weights, reduced mobility and curiosity, and/or visible or palpable tumors exceeding 15-20 mm) indicated that survival for additional 48 h was unlikely. Post-mortem examination confirmed the presence of tumors in all euthanized mice. Anti-PD1 antibody (Nivolumab) is a first-line drug for patients with metastatic renal cancer.**

| **Diet** | **P2** | **P3** | **P13** | **P14** | **P15** | **P16** | **P17** | **P18** | **P19** | **P20** | **P21** | **Anti PD1** | **Control** |
|---|---|---|---|---|---|---|---|---|---|---|---|---|---|
| **Mean survival (days)** | 47,7 | 73 | 54,6 | 40,3 | 50,3 | 38,6 | 47 | 40,3 | 44,3 | 55 | 43 | 43,2 | 30,3 |
| **Survival vs. Control** | 17,3 | 42,7 | 24,3 | 10 | 20 | 8,3 | 16,7 | 10 | 14 | 24,7 | 12,7 | 12,7 | - |

In all the active diets described in this invention, the levels of the amino acid methionine are less than or equal to 0.6%. Results shown in Table 4 indicate that the amount methionine should not be higher than 0.6% to achieve anticancer activity in mice with cancer. Diets P23 and P24, which contains 0.67% of methionine (0.5 of pure methionine + 0.17 of the methionine contained in 6 g of casein) did not have any antitumor activity. Diet P22 (which contains only the methionine provided by casein, i.e., 0.17%), however, showed a marked antitumor activity, even higher than the observed in mice treated with sunitinib (a first-line treatment for patients with metastatic renal cancer). Previous amino acid restricted diets (WO2017/053328) indicated that cysteine/cysteine should be eliminated or its levels reduced to obtain anticancer activity. However, this experiment shows that diet P22, which is rich in cysteine/cystine (0.5042%; 0.5 of pure cystine + 0,042 of the cysteine contained in 6 g of casein) was highly active, while diet P11, which contains a low amount of cysteine (only the 0.042 contained in 6 g of casein) had a low antitumor activity (P22 and P11 only differs in the amount of cysteine/cystine).

**Table 4: Survival of mice with renal cancer treated with several metabolic diets. Male BALB/cAnNRJ mice with renal cell carcinoma were treated with sunitinib (60 mg/kg/day, 28 days, oral administration), with one of the following diets: P22, P23, P24, P11, P9 (normal diet was replaced by one of these diets during 28 days), or left untreated (control, normal diet). Treatments started 7 days after the intraperitoneal injection of 150,000 Renca cancer cells. Mice that survived the 28-day treatment (with diets and sunitinib) were put on a normal diet for 10 days and then came back to treatment (diets or sunitinib) for additional 21 days (until day 66 after the inoculation of the cancer cells). At least four mice were included in each group Mice were euthanized by cervical dislocation when signs of disease progression were apparent; these signs (e.g., excessive gains and losses of body weights, reduced mobility and curiosity, and/or visible or palpable tumors exceeding 15-20 mm) indicated that survival for additional 48 h was unlikely. Post-mortem examination confirmed the presence of tumors in all euthanized mice. Sunitinib is a first-line drug for patients with metastatic renal cancer.**

| **Diet /treatment** | **P22** | **P23** | **P24** | **P11** | **P9** | **Control** | **Sunitinib** |
|---|---|---|---|---|---|---|---|
| **Mean survival (days)** | 52.5 | 30 | 29,75 | 33,25 | > 54.5 | 30,75 | 48 |
| **Survival improvement vs. Control** | 21,75 | -0,75 | -1 | 2,5 | > 23,75 | 0 | 17,25 |

In all the active diets described in this invention, the levels of the amino acid leucine are less than or equal to 10%. Several independent experiments indicate that controlling the levels of Leucine is important to increase antitumor activity in mice with renal cancer. In the experiment whose results are shown in Table 4, diet P11 (which contains only the leucine provided by casein, i.e., 0.54%) showed a moderate antitumor activity (mice lived 2.5 days more than untreated mice), while diet P9, which contains 3.04% of leucine (2.5 of pure leucine + 0.54 of the leucine contained in 6 g of casein) showed a marked antitumor effect (over 23.75 days more than untreated mice; one mouse is still alive and without any sign of disease). The anticancer activity of this diet (P9) was higher than the observed in mice treated with sunitinib (a first-line treatment for patients with metastatic renal cancer), which was 17,25 days.

The effect of controlling the levels of leucine on the anticancer activity of the diets is also observed in other cancers in vivo. For example, in mice with colon cancer, diet P10 (which contains 3.04% of leucine; 2.5 of pure leucine + 0.54 of the leucine contained in 6 g of casein) was much more active than diet P11 (which contains only the leucine provided by casein, i.e., 0.54%). Briefly, BALB/cAnNRJ mice with colon cancer were treated with Diet P10 (6 weeks), with diet P11 (6 weeks), with capecitabine (450 mg/kg/day, 7-day treatment + 7-day rest, until excessive toxicity or death, oral administration), or left untreated (control). At least three mice were included in each group. Treatments started 4 days after the intraperitoneal injection of 100,000 CT26.WT cancer cells. Mice were euthanized by cervical dislocation when signs of disease progression were apparent; these signs (e.g., excessive gains and losses of body weights, reduced mobility and curiosity, and/or visible or palpable tumors exceeding 15-20 mm) indicated that survival for additional 48 h was unlikely. Post-mortem examination confirmed the presence of tumors in all euthanized mice. Mice treated with capecitabine (a first-line treatment for patients with metastatic colon cancer) lived 4.5 days more than untreated mice, mice treated with diet P11 (0.54% leucine) lived 2.3 days more than untreated mice, and mice treated with diet P10 (3.04% leucine) lived > 44.1 days more than untreated mice (2 of the 7 mice used in this group are alive and without any sign of disease).

As discussed previously, caquexia and weigh loss are a common and important problem in patients with cancer. Because many of the diets shown in this invention are low in protein (and/or amino acids) and lipids, one might think that these diets will promote cachexia and weigh loss. In the experiment shown in Table 4, two mice lived long enough to compare weights when they were on diet P22 (6.5% protein and 1% fat) and a normal diet (21% protein and 7% fat). The weights of the two mice when diet P22 was initiated (day 7 after the inoculation of the cancer cells) were 29.2 g and 30.6 g. After 28 days (day 35) on diet P22 (low in protein and lipid), weights were 28.0 and 33.4, respectively. Mice came back to their normal diet (rich in protein and fat), and weights after 10 days (day 45) decreased to 24.2 g and 27.0 g. Then, diet P22 was reinitiated and 10 days later (day 55) weights increase to 28.0 g and 30.4 g. Mice died on day 63 and 83 (P22 treatment finished on day 66). These results clearly show that protein and lipid restriction does not necessarily promote cachexia. In fact, many of the diets shown in this invention induced anticancer effects without significantly affecting the weight of the animals and without inducing any apparent toxic effect.

Table 5 shows the survival improvements achieved with a variety of metabolic diets in mice with several types of cancer with respect to mice that did not receive any treatment (control). The survival improvements achieved with the drugs used in cancer patients are also shown.

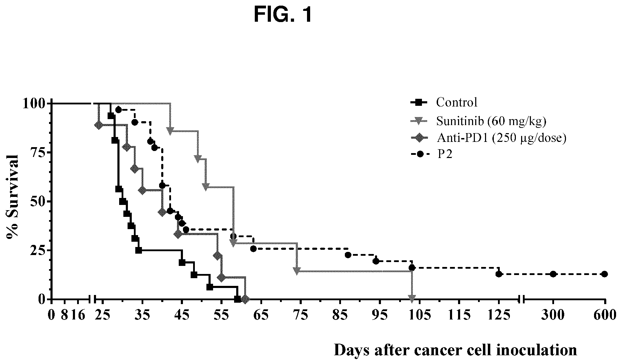

**Table 5: Anticancer activity of a variety of diets and several anticancer drugs in mice with different types of cancer Diet compositions (P1-P24) are shown in Tables 1 and 2 The experimental conditions are described in section "Mice and experimental in vivo conditions". The sign > indicates that one or several mice that received the treatment are still alive. The sign * indicates that the results are represented in Figures 1-4.**

| **Treatment** | **Type of cancer /General experimental conditions** | **Survival improvement vs untreated mice (mean, days)** |
|---|---|---|
| **Sunitinib** | **Renal cancer,** renca cells, 100.000, intraperitoneal injection (ip) | +20.4 |
| **Anti-PD1** | **Renal cancer,** renca cells, 100.000, intraperitoneal injection (ip) | +9.5 |
| **Anti-PD1** | **Lung cancer,** LLc1 cells, 2.000.000, tail vein injection (iv), C57BL/6J mice | -3.0 |
| **Capecitabine** | **Colon cancer,** CT26.WTcells, 10.000, intraperitoneal injection (ip) | +7.6 |
| **Capecitabine** | **Colon cancer,** CT26.WTcells, 10.000, tail vein injection (iv) | 0.0 |
| **Capecitabine** | **Breast cancer,** 4T1 cells, 100.000, tail vein injection (iv) | +3.7 |
| **Cisplatin** | **Breast cancer,** 4T1 cells, 100.000, tail vein injection (iv) | + 4.7 |
| **Cisplatin** | **Melanoma,** B16-F10 cells, 500.000, tail vein injection (iv), C57BL/6J mice | + 3.5 |
| **P1** | **Renal cancer**, renca cells, 100.000, intraperitoneal injection (ip) | + 6.5 |
| **P1** | **Breast cancer**, 4T1 cells, 100.000, tail vein injection (iv) | + 4.4 |
| **P1** | **Colon cancer**, CT26.WTcells, 100.000, intraperitoneal injection (ip) | + 9.4 |
| **P2** | **Renal cancer**, renca cells, 100.000, intraperitoneal injection (ip) | > + 76.8 * |
| **P3** | **Renal cancer**, renca cells, 100.000, intraperitoneal injection (ip) | + 42.7 |
| **P4** | **Breast cancer**, 4T1 cells, 100.000, tail vein injection (iv) | + 11 9 |
| **P5** | **Breast cancer**, 4T1 cells, 100.000, tail vein injection (iv) | + 33.7 |
| **P6** | **Breast cancer**, 4T1 cells, 100.000, tail vein injection (iv) | + 17.7 * |
| **P6** | **Colon cancer**, CT26.WTcells, 10.000, intraperitoneal injection (ip) | + 4.3 |
| **P6** | **Colon cancer**, CT26.WTcells, 10.000, tail vein injection (iv) | + 44.7* |
| **P6** | **Lung cancer**, LLc1 cells, 2.000.000, tail vein injection (iv), C57BL/6J mice | + 3.7 |
| **P6** | **Melanoma**, B16-F10 cells, 500.000, tail vein injection (iv), C57BL/6J mice | + 4.1 |
| **P7** | **Breast cancer**, 4T1 cells, 100.000, tail vein injection (iv) | + 25,9 |
| **P8** | **Breast cancer**, 4T1 cells, 100.000, tail vein injection (iv) | > + 34.0 * |
| **P9** | **Renal cancer**, renca cells, 150.000, intraperitoneal injection (ip) | >+ 23.75 |
| **P10** | **Colon cancer**, CT26.WTcells, 10.000, intraperitoneal injection (ip) | >+ 44.1 * |
| **P10** | **Colon cancer**, CT26.WTcells, 10.000, tail vein injection (iv) | + 9.0 |
| **P11** | **Colon cancer**, CT26.WTcells, 10.000, tail vein injection (iv) | + 7.5 |
| **P11** | **Colon cancer**, CT26.WTcells, 10.000, intraperitoneal injection (ip) | + 2.3 |
| **P11** | **Renal cancer**, renca cells, 150.000, intraperitoneal injection (ip) | + 2.5 |
| **P12** | **Renal cancer**, renca cells, 150.000, intraperitoneal injection (ip) | >+ 9.7 |
| **P13** | **Renal cancer**, renca cells, 100.000, intraperitoneal injection (ip) | + 24.3 |
| **P14** | **Renal cancer**, renca cells, 100.000, intraperitoneal injection (ip) | + 100 |
| **P15** | **Renal cancer**, renca cells, 100.000, intraperitoneal injection (ip) | + 20.0 |
| **P16** | **Renal cancer**, renca cells, 100.000, intraperitoneal injection (ip) | + 8.3 |
| **P17** | **Renal cancer**, renca cells, 100.000, intraperitoneal injection (ip) | + 16.7 |
| **P18** | **Renal cancer**, renca cells, 100.000, intraperitoneal injection (ip) | + 100 |
| **P19** | **Renal cancer**, renca cells, 100.000, intraperitoneal injection (ip) | + 14.0 |
| **P20** | **Renal cancer**, renca cells, 100.000, intraperitoneal injection (ip) | + 24,7 |
| **P21** | **Renal cancer**, renca cells, 100.000, intraperitoneal injection (ip) | + 12.7 |
| **P22** | **Renal cancer**, renca cells, 150.000, intraperitoneal injection (ip) | + 21.7 |
| **P23** | **Renal cancer**, renca cells, 150.000, intraperitoneal injection (ip) | -0.7 |
| **P24** | **Renal cancer**, renca cells, 150.000, intraperitoneal injection (ip) | -1.0 |

### Anticancer activity of diet P2, Sunitinib and Anti-PD1 in mice with renal cancer

Figure 1 shows survival of male BALB/cAnNRJ mice with renal cell carcinoma treated with sunitinib (60 mg/kg/day, 28 days, oral administration), with anti-PD1 antibody (250µg administered intraperitoneally on days 8, 12, 16 and 20), with diet P2 (28 days; normal diet was replaced by P2 diet), or left untreated (control, normal diet). Treatments started 8 days after the intraperitoneal injection of 100,000 Renca cancer cells. Mice were euthanized by cervical dislocation when signs of disease progression were apparent; these signs (e.g., excessive gains and losses of body weights, reduced mobility and curiosity, and/or visible or palpable tumors exceeding 15-20 mm) indicated that survival for additional 48 h was unlikely. Post-mortem examination confirmed the presence of tumors in all euthanized mice. Data were averaged from at least two independent experiments; mean survival were 35,2days for control (n= 16), 62,2 days for sunitinib (n=7), 41,9 days for anti-PD1 (n=9), and >112,0 days for diet P2 (n=31). Several mice treated with diet P2 are alive and without any sign of disease. Sunitinib and anti-PD1 antibody (Nivolumab) are first-line drugs for patients with metastatic renal cancer.

### Anticancer activity of diet P10 and capecitabine in mice with colon cancer (intraperitoneal model)

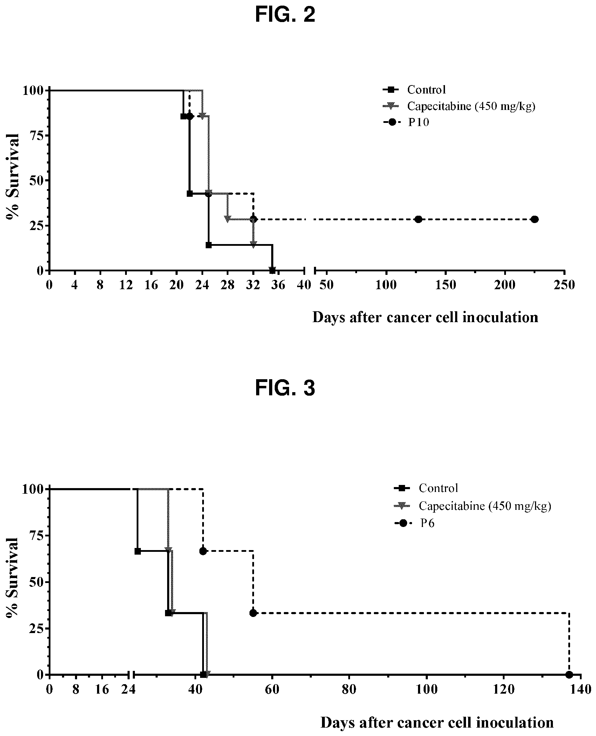

Figure 2 shows survival of female BALB/cAnNRJ mice with colon cancer treated with capecitabine (450 mg/kg/day, 7-day treatment + 7-day rest, until excessive toxicity or death, oral administration), with Diet P10 (6 weeks), or left untreated (control). Treatments started 4 days after the intraperitoneal injection of 100,000 CT26.WT cancer cells. Mice were euthanized by cervical dislocation when signs of disease progression were apparent; these signs (e.g., excessive gains and losses of body weights, reduced mobility and curiosity, and/or visible or palpable tumors exceeding 15-20 mm) indicated that survival for additional 48 h was unlikely. Post-mortem examination confirmed the presence of tumors in all euthanized mice. Data were averaged from two independent experiments; mean survival were 24.6 days for control (n= 7), 27.7 days for capecitabine (n=7), and >68,7 days for diet P10 (n=7). Two mice treated with diet P10 are alive and without any sign of disease. Capecitabine (a pro-drug of 5-Fluorouracil) is a first-line treatment for patients with metastatic colon cancer.

### Anticancer activity of diet P6 and capecitabine in mice with colon cancer (intravenous model)

Figure 3 shows survival of female BALB/cAnNRJ mice with colon cancer treated with capecitabine (450 mg/kg/day, 7-day treatment + 7-day rest until excessive toxicity or death, oral administration), with diet P6 (28 days), or left untreated (control). Treatments started 4 days after the tail vein injection of 100,000 CT26.WT cancer cells. Mice were euthanized by cervical dislocation when signs of disease progression were apparent; these signs (e.g., respiratory distress, excessive gains and losses of body weights, reduced mobility and curiosity, and/or visible or palpable tumors exceeding 15-20 mm) indicated that survival for additional 48 h was unlikely. Post-mortem examination confirmed the presence of tumors in all euthanized mice, mainly in the lungs. Mean survival were 33,3 days for control (n= 3), 36,7 days for capecitabine (n=3), and 78 days for diet P6 (n=3).

### Anticancer activity of diet P6, diet P8, cisplatin and capeciabine in mice with triple negative breast cancer (intravenous model)

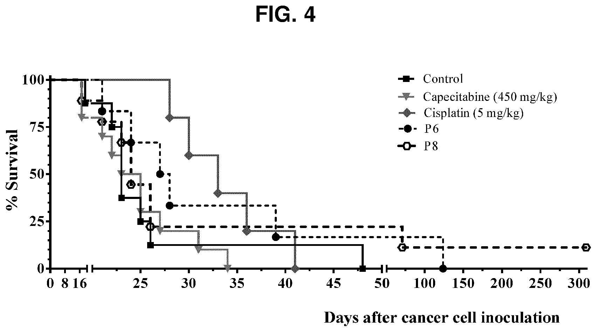

Figure 4 shows survival of female BALB/cAnNRJ mice with triple negative breast cancer treated with capecitabine (450 mg/kg/day, 7-day treatment + 7-day rest until excessive toxicity or death, oral administration), with cisplatin (5 mg/kg weekly during 4 weeks, intraperitoneal injection), with diet P6 (28 days), with diet P8 (28 days), or left untreated (control). Treatments started 8 days after the tail vein injection of 100,000 4T1 cancer cells. Mice were euthanized by cervical dislocation when signs of disease progression were apparent; these signs (e.g., respiratory distress, excessive gains and losses of body weights, reduced mobility and curiosity, and/or visible or palpable tumors exceeding 15-20 mm) indicated that survival for additional 48 h was unlikely. Post-mortem examination confirmed the presence of tumors in all euthanized mice, mainly in the lungs. Mean survival were 26,1 days for control (n= 8), 24,2 days for capecitabine (n=10; two mice died prematurely by excessive drug toxicity), 33,6 days for cisplatin (n=5), 43,8 days for diet P6 (n=6), and 60,1 days for diet P8 (n=9). One mouse treated with diet P8 is still alive (it developed signs of disease twice, but they disappeared with 4 additional weeks on diet P8). Capecitabine (a pro-drug of 5-Fluorouracil) is a first-line treatment for patients with triple-negative breast cancer. Cisplatin is a drug widely used in many types of cancer.

### In vitro anticancer activity of amino-acid-restricted compositions

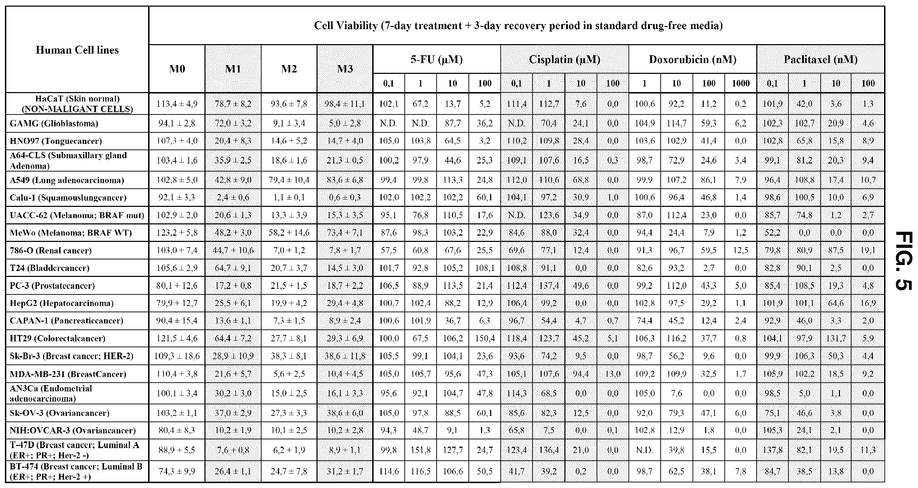

Cell culture media M1 (equivalent to diet P1), M2 (equivalent to diet P2) and M3 (equivalent to diet P3) were prepared to evaluate their cytotoxicity and selectivity towards cancer cells. Medium M0, which contains all the amino acids, was also prepared and tested under the same experimental conditions. Media composition are shown in Table 6. These media were tested in human normal skin cells with high proliferative rates (HaCaT) and in 20 types of human cancer cells representing the most common types of cancer. Four commonly used anticancer drugs (representing the main types of chemotherapy) were also tested in the 21 human cell lines to compare the selective anticancer activity of the amino acid restricted media with that of the standard anticancer drugs. Data in Figure 5 shows that normal cells exposed to the amino acid restricted media grew relatively well (cell viabilities were between 78 and 98%), while most types of cancer cells were highly affected when cultured in these media (cell viabilities were low). However, none of the anticancer drugs at any dose induced marked reductions in the viability of cancer cells without reducing the viability of the normal cells. These results show that the amino acid restricted media are more selective towards cancer cells than the standard anticancer drugs used in patients.

**Table 6. Composition of cell culture media M0, M1, M2 and M3.**

| | **M0** | **M1** | **M2** | **M3** |
|---|---|---|---|---|
| Fetal bovine serum | 10% | 10% | 10% | 10% |
| Penicillin/streptomycin | 1% | 1% | 1% | 1% |

| **Compound** | **mg/L** | **mg/L** | **mg/L** | **mg/L** |
|---|---|---|---|---|
| Calciumchloridedihydrate | 265 | 265 | 265 | 265 |
| Magnesium sulfate | 98 | 98 | 98 | 98 |
| Ferric (III) nitrate | 0,1 | 0,1 | 0,1 | 0,1 |
| Potassiumchloride | 400 | 400 | 400 | 400 |
| Sodiumphosphatemonobasic | 109 | 109 | 109 | 109 |
| Sodiumchloride | 6400 | 6400 | 6400 | 6400 |
| D-glucose | 4500 | 4500 | 4500 | 4500 |
| Cholinechloride | 4 | 4 | 4 | 4 |
| D -Pantothenic acid hemicalcium salt | 4 | 4 | 4 | 4 |
| Folicacid | 4 | 4 | 4 | 4 |
| Nicotinamide | 4 | 4 | 4 | 4 |
| Pyridoxinehydrochloride | 4 | 4 | 4 | 4 |
| Thiaminehydrochlorie | 4 | 4 | 4 | 4 |
| Myo-inositol | 7,2 | 7,2 | 7,2 | 7,2 |
| D-biotin | 0,2 | 0,2 | 0,2 | 0,2 |
| Riboflavin | 0,4 | 0,4 | 0,4 | 0,4 |
| vitamin B12 | 0,005 | 0,005 | 0,005 | 0,005 |
| Sodiumbicarbonate | 3700 | 3700 | 3700 | 3700 |
| L-phenylalanine | 192 | 192 | 192 | 192 |
| L-histidine | 76 | 76 | 76 | 76 |
| L-lysine | 235 | 235 | 235 | 235 |
| L-threonine | 160 | 160 | 160 | 160 |
| L-Isoleucine | 95 | 95 | 95 | 95 |
| L-valine | 235 | 235 | 235 | 235 |
| L-leucine | 533 | 533 | 533 | 533 |
| L-Tryptophan | 21 | 21 | 21 | 21 |
| L-methionine | 53 | 53 | 53 | 53 |
| L-glutamine | 533 | 533 | 533 | 533 |
| L-arginine | 133 | - | 133 | 133 |
| Glycine | 89 | - | 89 | 89 |
| L-alanine | 89 | - | 89 | 89 |
| L-asparticacid | 178 | - | 178 | 178 |
| L-serine | 42 | - | - | - |
| L-tyrosine | 89 | - | - | 268 |
| L-Cystinedihydrochloride | 63 | - | - | - |
| L-asparagine-1-hydrate | 50 | - | - | - |
| L-glutamicacid | 20 | - | - | - |
| L-proline | 20 | - | - | - |

### REFERENCES:

1. Siegel RL, Miller KD, Jemal A. Cancer statistics, 2020. CA Cancer J Clin. 2020; 70(1):7-30.
2. Lopez-Lazaro M. Selective Amino Acid Restriction Therapy (SAART): a non-pharmacological strategy against all types of cancer cells. Oncoscience. 2015; 2(10): 857-866.
3. Gottesman MM. Mechanisms of cancer drug resistance. Annu Rev Med. 2002; 53: 615-27.
4. Hall MD, Handley MD, Gottesman MM. Is resistance useless? Multidrug resistance and collateral sensitivity. Trends Pharmacol Sci. 2009; 30(10): 546-556.
5. Lopez-Lazaro M. The stem cell division theory of cancer. Crit Rev Oncol Hematol. 2018; 123: 95-113.
6. Lopez-Lazaro M. The migration ability of stem cells can explain the existence of cancer of unknown primary site. Rethinking metastasis. Oncoscience. 2015; 2(5): 467-475.
7. Lopez-Lazaro M. Cancer arises from stem cells: opportunities for anticancer drug discovery. Drug Discov Today. 2015; doi: 10.1016/j.drudis.2015.09.006. [Epub ahead of print]
8. Dean M, Fojo T, Bates S. Tumour stem cells and drug resistance. Nat Rev Cancer. 2005; 5(4): 275-284.
9. Dean M. ABC transporters, drug resistance, and cancer stem cells. J Mammary Gland Biol Neoplasia. 2009; 14(1): 3-9.
10. Kaiser J. The cancer stem cell gamble. Science. 2015; 347(6219): 226-229.
11. Blagosklonny MV. Cancer stem cell and cancer stemloids: from biology to therapy. Cancer Biol Ther. 2007; 6(11): 1684-1690.
12. Gibbs WW. Untangling the roots of cancer. Sci Am. 2003; 289(1): 56-65.
13. Gordon DJ, Resio B, Pellman D. Causes and consequences of aneuploidy in cancer. Nat Rev Genet. 2012; 13(3): 189- 203.
14. Stratton MR. Exploring the genomes of cancer cells: progress and promise. Science. 2011; 331(6024): 1553- 1558.
15. Forbes SA, Beare D, Gunasekaran P, Leung K, Bindal N, Boutselakis H, Ding M, Bamford S, Cole C, Ward S, Kok CY, Jia M, De T et al. COSMIC: exploring the world's knowledge of somatic mutations in human cancer. Nucleic Acids Res. 2015; 43(Database issue): D805-D811.
16. Lopez-Lazaro M. Cancer etiology: Variation in cancer risk among tissues is poorly explained by the number of gene mutations. Genes Chromosomes Cancer. 2018 Jun;57(6):281-293.
17. McGranahan N, Swanton C. Biological and therapeutic impact of intratumor heterogeneity in cancer evolution. Cancer Cell. 2015; 27(1): 15-26.
18. Stewart DJ, Whitney SN, Kurzrock R. Equipoise lost: ethics, costs, and the regulation of cancer clinical research. J Clin Oncol. 2010; 28(17): 2925-2935.
19. Kantarjian H, Stewart DJ, Zwelling L. Cancer research in the United States: dying by a thousand paper cuts. Cancer. 2013; 119(21): 3742-3745.
20. Kantarjian H, Zwelling L. Cancer drug prices and the free-market forces. Cancer. 2013; 119(22): 3903-3905.
21. Weinberg RA. Coming full circle-from endless complexity to simplicity and back again. Cell. 2014; 157(1): 267-271.
22. Hanahan D. Rethinking the war on cancer. Lancet. 2014; 383(9916): 558-563.
23. Toyama BH, Hetzer MW. Protein homeostasis: live long, won't prosper. Nat Rev Mol Cell Biol. 2013; 14(1): 55-61.
24. Yen HC, Xu Q, Chou DM, Zhao Z, Elledge SJ. Global protein stability profiling in mammalian cells. Science. 2008; 322(5903): 918-923.
25. Scott L, Lamb J, Smith S, Wheatley DN. Single amino acid (arginine) deprivation: rapid and selective death of cultured transformed and malignant cells. Br J Cancer. 2000; 83(6): 800-810.
26. Rohrig F, Schulze A. The multifaceted roles of fatty acid synthesis in cancer. Nat Rev Cancer. 2016; 16(11):732-749.
27. McCarthy DO. Rethinking nutritional support for persons with cancer cachexia. Biol Res Nurs. 2003; 5: 3-17.
28 Kern KA, Norton JA. Cancer cachexia.JPEN J Parenter Enteral Nutr. 1988;12(3):286-298.
29. Bozzetti F, Mariani L. Defining and classifying cancer cachexia: a proposal by the SCRINIO Working Group. JPEN J Parenter Enteral Nutr. 2009;33(4):361-367.

## Claims

1. Artificial diet composition for use in the treatment and/or prevention of cancer comprising, (based on the total weight of the dry ingredients composition) :
- from 4 to 40% of a mixture of amino acids,
- from 0 - 25% lipids,
- from 40 - 95% carbohydrates,
- from 1 to 5% of a mixture of vitamins and minerals, and
- from 0 to 1% choline,
**characterized in that** Leucine is present in the composition as part of the mixture of amino acids in an amount of from 0.5- 6% by weight with respect to the total weight of the dry ingredients composition and Methionine is present in the composition as part of the mixture of amino acids in an amount of from 0,1 - 0,6% by weight with respect to the total weight of the dry ingredients composition.

2. Artificial diet composition for use according to claim 1, **characterized in that** the mixture of amino acids is a mixture of essential and non-essential amino acids, selected from the group consisting of: leucine, isoleucine, valine, methionine, lysine, phenylalanine, tryptophan, threonine, histidine, asparagine, alanine, arginine, aspartic acid, cysteine/cystine, glutamic acid, glutamine, proline, glycine, tyrosine, serine and mixtures thereof.

3. Artificial diet composition for use according to any previous claim, **characterized in that** the amino acids are in the free form, salt form, ester form and/or in the form of a peptide, polypeptide or protein.

4. Artificial diet composition for use according to any previous claim, **characterized in that** the amino acids present in the composition are a combination of amino acids in the free form and as a protein.

5. Artificial diet composition for use according to claim 4, **characterized in that** the protein is casein.

6. Artificial diet composition for use according to any preceding claim, **characterized in that** the lipid ingredient is present in an amount of from 0 - 14% by weight with respect to the total weight of the dry composition.

7. Artificial diet composition for use according to claim 6, **characterized in that** the lipid ingredient is selected from any edible vegetable or animal oils, selected from: olive oil, coconut oil, salmon oil, corn oil, soybean oil, canola oil, rapeseed oil, sunflower oil, linseed oil, rice oil, safflower oil, cottonseed oil, palm oil, castor seed oil, peanut oil, wheat oil, pumpkin seed oil, poppy seed oil, hemp oil, pomegranate seed oil, cod oil, herring oil, whale oil, seal oil, margarine, butter, lard, tallow, and mixtures thereof.

8. Artificial diet composition for use according to claims 6 and 7, **characterized in that** the one or more lipid ingredient is selected from the group consisting of: olive oil, coconut oil and salmon oil.

9. Artificial diet composition for use according to any preceding claim, **characterized in that** the carbohydrates can be selected from the group consisting of: saccharose, cellulose, starch and mixtures thereof.

10. Artificial diet composition for use according to any previous claim, **characterized in that** it is in a form suitable for oral administration.

11. Artificial diet composition for use according to claim 10, **characterized in that** it is in a solid, semisolid or liquid form and is selected from: dry powder, shake, liquid concentrates, drink ready to drink, chilled or shelf stable beverage, soup, paste, puree, nutritional bar.

12. Artificial diet composition for use according to any previous claim, **characterized in that** treatment of cancer comprises: renal cancer, lung cancer, colon cancer, breast cancer, melanoma, ovarian cancer, prostate cancer, pancreatic cancer, liver cancer, endometrial cancer, cervical cancer, bladder cancer, esophageal cancer, gastric cancer, head and neck cancers, leukemia, lymphomas, non-melanoma skin cancers, sarcomas, central nervous system cancers, testicular cancer, thyroid cancer and cancer of unknown primary site.

13. Artificial diet composition for use according to any preceding claim, **characterized in that** such treatment comprises taking the artificial diet composition as a replacement of meal to provide the necessary daily nutritional requirements of the subject.

14. Artificial diet composition for use according to any preceding claim, **characterized in that** treatment comprises treatment cycles of two to twelve weeks with a four to six daily doses.

15. Artificial diet composition for use according to any previous claim, **characterized in that** it further comprises water or a water based carrier.

16. A pharmaceutical composition comprising the artificial diet composition for use according to any of claims 1 to 15 together with a pharmaceutical acceptable carrier or vehicle, for use in the treatment of cancer.

17. A pharmaceutical composition for use according to claim 16, **characterized in that** treatment of cancer comprises: renal cancer, lung cancer, colon cancer, breast cancer, melanoma, ovarian cancer, prostate cancer, pancreatic cancer, liver cancer, endometrial cancer, cervical cancer, bladder cancer, esophageal cancer, gastric cancer, head and neck cancers, leukemia, lymphomas, non-melanoma skin cancers, sarcomas, central nervous system cancers, testicular cancer, thyroid cancer and cancer of unknown primary site.

18. A pharmaceutical composition for use according to claims 16 to 17, **characterized in that** treatment comprises co-administration of the pharmaceutical composition together with any type of drug therapy, including cytotoxic chemotherapy drugs such as alkylating agents (e.g., cisplatin, carboplatin, oxaliplatin, cyclophosphamide, temozolomide, hydroxyurea, etc), antimetabolites (e.g., fluorouracil, capecitabine, gemcitabine, methotrexate, cytarabine etc), mitotic inhibitors (e.g., paclitaxel, docetaxel, cabacitaxel, vincristine, vinblastine, vinorelbine, vindesine, etc), topoisomerase inhibitors (e.g., etoposide, teniposide, irinotecan, topotecan, doxorubicin, epirrubicin, etc), hormonal therapy (e.g., antiestrogens such as tamoxifen, aromatase inhibitors such as anastrozole, LHRH agonists such as goserelin, antiandrogens such as abiraterone and flutamide, corticosteroids such as dexamethasone and prednisone, etc), immunotherapies (e.g., anti-PD1 such as nivolumab, anti-PDL1 such as avelumab, anti-CTLA4 such as ipilimumab, cytokines such as interleukin-2 and interferon, etc), targeted therapies (e.g., anti-VEGF agents such as bevacizumab, anti-VEGFR such as sunitinib and sorafenib, anti-EGFR such as cetuximab or erlotinib, anti-HER2 such as trastuzumab, anti-PARP such as olaparib, anti-BRAF such as vemurafenib, etc), and any other anticancer drug as well as mixtures thereof.

19. A pharmaceutical composition for use according to claim 18, **characterized in that** such co-administration comprises sequential, concomitant or simultaneous administration of active ingredients.

20. A pharmaceutical composition for use according to claims 16 to 19, **characterized in that** treatment comprises administering to a subject in need thereof of an effective amount of the pharmaceutical composition during surgery and/or radiotherapy treatment.

## Patentansprüche

1. Künstliche Diätzusammensetzung zur Verwendung bei der Behandlung und/oder Vorbeugung von Krebs, umfassend (bezogen auf das Gesamtgewicht der Trockenbestandteilzusammensetzung):
- 4 bis 40 % einer Mischung von Aminosäuren,
- 0-25 % Lipide,
- 40-95 % Kohlenhydrate,
- 1 bis 5 % einer Mischung aus Vitaminen und Mineralstoffen und
- 0 bis 1 % Cholin,
**dadurch gekennzeichnet, dass** Leucin in der Zusammensetzung als Teil der Aminosäuremischung in einer Menge von 0,5-6 Gew.-%, bezogen auf das Gesamtgewicht der Trockenbestandteilzusammensetzung, vorhanden ist und Methionin in der Zusammensetzung als Teil der Aminosäuremischung in einer Menge von 0,1-0,6 Gew.-%, bezogen auf das Gesamtgewicht der Trockenbestandteilzusammensetzung, vorhanden ist.

2. Künstliche Diätzusammensetzung zur Verwendung gemäß Anspruch 1, **dadurch gekennzeichnet, dass** die Aminosäuremischung eine Mischung aus essentiellen und nicht essentiellen Aminosäuren ist, ausgewählt aus der Gruppe, bestehend aus: Leucin, Isoleucin, Valin, Methionin, Lysin, Phenylalanin, Tryptophan, Threonin, Histidin, Asparagin, Alanin, Arginin, Asparaginsäure, Cystein/Cystin, Glutaminsäure, Glutamin, Prolin, Glycin, Tyrosin, Serin und Mischungen davon.

3. Künstliche Diätzusammensetzung zur Verwendung gemäß einem der vorhergehenden Ansprüche, **dadurch gekennzeichnet, dass** die Aminosäuren in freier Form, Salzform, Esterform und/oder in Form eines Peptids, Polypeptids oder Proteins vorliegen.

4. Künstliche Diätzusammensetzung zur Verwendung gemäß einem der vorhergehenden Ansprüche, **dadurch gekennzeichnet, dass** die in der Zusammensetzung vorhandenen Aminosäuren eine Kombination aus Aminosäuren in freier Form und als Protein sind.

5. Künstliche Diätzusammensetzung zur Verwendung gemäß Anspruch 4, **dadurch gekennzeichnet, dass** das Protein Casein ist.

6. Künstliche Diätzusammensetzung zur Verwendung gemäß einem der vorhergehenden Ansprüche, **dadurch gekennzeichnet, dass** der Lipidbestandteil in einer Menge von 0-14 Gew.-%, bezogen auf das Gesamtgewicht der trockenen Zusammensetzung, vorhanden ist.

7. Künstliche Diätzusammensetzung zur Verwendung gemäß Anspruch 6, **dadurch gekennzeichnet, dass** der Lipidbestandteil aus beliebigen essbaren pflanzlichen oder tierischen Ölen ausgewählt ist, die aus: Olivenöl, Kokosnussöl, Lachsöl, Maisöl, Sojaöl, Canolaöl, Rapsöl, Sonnenblumenöl, Leinöl, Reisöl, Distelöl, Baumwollsamenöl, Palmöl, Rizinusöl, Erdnussöl, Weizenöl, Kürbiskernöl, Mohnöl, Hanföl, Granatapfelkernöl, Dorschtran, Heringsöl, Walöl, Robbenöl, Margarine, Butter, Schweineschmalz, Talg und Mischungen davon ausgewählt sind.

8. Künstliche Diätzusammensetzung zur Verwendung gemäß den Ansprüchen 6 und 7, **dadurch gekennzeichnet, dass** der eine oder die mehreren Lipidbestandteile aus der Gruppe ausgewählt sind, die aus Olivenöl, Kokosnussöl und Lachsöl besteht.

9. Künstliche Diätzusammensetzung zur Verwendung gemäß einem der vorhergehenden Ansprüche, **dadurch gekennzeichnet, dass** die Kohlenhydrate aus der Gruppe ausgewählt sein können, die aus Saccharose, Zellulose, Stärke und Mischungen davon besteht.

10. Künstliche Diätzusammensetzung zur Verwendung gemäß einem der vorhergehenden Ansprüche, **dadurch gekennzeichnet, dass** sie in einer für die orale Verabreichung geeigneten Form vorliegt.

11. Künstliche Diätzusammensetzung zur Verwendung nach Anspruch 10, **dadurch gekennzeichnet, dass** sie in fester, halbfester oder flüssiger Form vorliegt und aus: Trockenpulver, Shake, flüssigen Konzentraten, trinkfertigem Getränk, gekühltem oder haltbarem Getränk, Suppe, Paste, Püree, Ernährungsriegel ausgewählt ist.

12. Künstliche Diätzusammensetzung zur Verwendung gemäß einem der vorhergehenden Ansprüche, **dadurch gekennzeichnet, dass** die Behandlung von Krebs folgende Erkrankungen umfasst: Nierenkrebs, Lungenkrebs, Dickdarmkrebs, Brustkrebs, Melanom, Eierstockkrebs, Prostatakrebs, Bauchspeicheldrüsenkrebs, Leberkrebs, Endometriumkrebs, Gebärmutterhalskrebs, Blasenkrebs, Speiseröhrenkrebs, Magenkrebs, Kopf- und Halskrebs, Leukämie, Lymphome, nicht melanozytären Hautkrebs, Sarkome, Krebserkrankungen des zentralen Nervensystems, Hodenkrebs, Schilddrüsenkrebs und Krebs mit unbekanntem Primärtumor.

13. Künstliche Diätzusammensetzung zur Verwendung gemäß einem der vorhergehenden Ansprüche, **dadurch gekennzeichnet, dass** eine solche Behandlung die Einnahme der künstlichen Diätzusammensetzung als Mahlzeitenersatz zur Deckung des notwendigen täglichen Nährstoffbedarfs des Subjekts umfasst.

14. Künstliche Diätzusammensetzung zur Verwendung gemäß einem der vorhergehenden Ansprüche, **dadurch gekennzeichnet, dass** die Behandlung Behandlungszyklen von zwei bis zwölf Wochen mit vier bis sechs täglichen Dosen umfasst.

15. Künstliche Diätzusammensetzung zur Verwendung gemäß einem der vorhergehenden Ansprüche, **dadurch gekennzeichnet, dass** sie ferner Wasser oder einen Träger auf Wasserbasis umfasst.

16. Pharmazeutische Zusammensetzung, die die künstliche Diätzusammensetzung zur Verwendung gemäß einem der Ansprüche 1 bis 15 zusammen mit einem pharmazeutisch annehmbaren Träger oder Vehikel zur Verwendung bei der Behandlung von Krebs umfasst.

17. Pharmazeutische Zusammensetzung zur Verwendung gemäß Anspruch 16, **dadurch gekennzeichnet, dass** die Behandlung von Krebs folgende Erkrankungen umfasst: Nierenkrebs, Lungenkrebs, Dickdarmkrebs, Brustkrebs, Melanom, Eierstockkrebs, Prostatakrebs, Bauchspeicheldrüsenkrebs, Leberkrebs, Endometriumkrebs, Gebärmutterhalskrebs, Blasenkrebs, Speiseröhrenkrebs, Magenkrebs, Kopf- und Halskrebs, Leukämie, Lymphome, nicht melanozytären Hautkrebs, Sarkome, Krebserkrankungen des zentralen Nervensystems, Hodenkrebs, Schilddrüsenkrebs und Krebs mit unbekanntem Primärtumor.

18. Pharmazeutische Zusammensetzung zur Verwendung gemäß den Ansprüchen 16 bis 17, **dadurch gekennzeichnet, dass** die Behandlung die gemeinsame Verabreichung der pharmazeutischen Zusammensetzung zusammen mit jeder Art von medikamentöser Therapie umfasst, darunter zytotoxische Chemotherapeutika wie Alkylierungsmittel (z. B. Cisplatin, Carboplatin, Oxaliplatin, Cyclophosphamid, Temozolomid, Hydroxyharnstoff usw.), Antimetaboliten (z. B. Fluoruracil, Capecitabin, Gemcitabin, Methotrexat, Cytarabin usw.), Mitosehemmer (z. B. Paclitaxel, Docetaxel, Cabacitaxel, Vincristin, Vinblastin, Vinorelbin, Vindesin usw.), Topoisomerasehemmer (z. B. Etoposid, Teniposid, Irinotecan, Topotecan, Doxorubicin, Epirubicin usw.), Hormontherapie (z. B. Antiöstrogene wie Tamoxifen, Aromatasehemmer wie Anastrozol, LHRH-Agonisten wie Goserelin, Antiandrogene wie Abirateron und Flutamid, Kortikosteroide wie Dexamethason und Prednison usw.), Immuntherapien (z. B. Anti-PD1 wie Nivolumab, Anti-PDL1 wie Avelumab, Anti-CTLA4 wie Ipilimumab, Zytokine wie Interleukin-2 und Interferon usw.), zielgerichtete Therapien (z. B. Anti-VEGF-Wirkstoffe wie Bevacizumab, Anti-VEGFR wie Sunitinib und Sorafenib, Anti-EGFR wie Cetuximab oder Erlotinib, Anti-HER2 wie Trastuzumab, Anti-PARP wie Olaparib, Anti-BRAF wie Vemurafenib usw.) und jedes andere Krebsmedikament sowie Mischungen davon.

19. Pharmazeutische Zusammensetzung zur Verwendung gemäß Anspruch 18, **dadurch gekennzeichnet, dass** eine derartige gemeinsame Verabreichung eine aufeinander folgende, begleitende oder gleichzeitige Verabreichung der Wirkstoffe umfasst.

20. Pharmazeutische Zusammensetzung zur Verwendung gemäß den Ansprüchen 16 bis 19, **dadurch gekennzeichnet, dass** die Behandlung die Verabreichung einer wirksamen Menge der pharmazeutischen Zusammensetzung während einer Operation und/oder einer Strahlentherapie an einen Patienten, der diese benötigt, umfasst.

## Revendications

1. Composition alimentaire artificielle destinée à être utilisée dans le traitement et/ou la prévention d'un cancer comprenant, (sur la base du poids total de la composition d'ingrédients secs) :
- de 4 à 40 % d'un mélange d'acides aminés,
- de 0 à 25 % de lipides,
- de 40 à 95 % de glucides,
- de 1 à 5 % d'un mélange de vitamines et de minéraux et
- de 0 à 1 % de choline,
**caractérisée en ce que** de la leucine est présente dans la composition en tant que partie du mélange d'acides aminés en une quantité allant de 0,5 à 6 % en poids par rapport au poids total de la composition d'ingrédients secs et de la méthionine est présente dans la composition en tant que partie du mélange d'acides aminés en une quantité de 0,1 à 0,6 % en poids par rapport au poids total de la composition d'ingrédients secs.

2. Composition alimentaire artificielle destinée à être utilisée selon la revendication 1, **caractérisée en ce que** le mélange d'acides aminés est un mélange d'acides aminés essentiels et non essentiels, choisis dans le groupe composé de : la leucine, l'isoleucine, la valine, la méthionine, la lysine, la phénylalanine, le tryptophane, la thréonine, l'histidine, l'asparagine, l'alanine, l'arginine, l'acide aspartique, la cystéine/cystine, l'acide glutamique, la glutamine, la proline, la glycine, la tyrosine, la sérine et les mélanges de ceux-ci.

3. Composition alimentaire artificielle destinée à être utilisée selon une quelconque revendication précédente, **caractérisée en ce que** les acides aminés sont sous la forme libre, la forme de sel, la forme d'ester et/ou sous la forme d'un peptide, d'un polypeptide ou d'une protéine.

4. Composition alimentaire artificielle destinée à être utilisée selon une quelconque revendication précédente, **caractérisée en ce que** les acides aminés présents dans la composition sont une combinaison d'acides aminés sous la forme libre et en tant que protéine.

5. Composition alimentaire artificielle destinée à être utilisée selon la revendication 4, **caractérisée en ce que** la protéine est la caséine.

6. Composition alimentaire artificielle destinée à être utilisée selon une quelconque revendication précédente, **caractérisée en ce que** l'ingrédient lipidique est présent en une quantité allant de 0 à 14 % en poids par rapport au poids total de la composition sèche.

7. Composition alimentaire artificielle destinée à être utilisée selon la revendication 6, **caractérisée en ce que** l'ingrédient lipidique est choisi parmi de quelconques huiles végétales ou animales comestibles, choisies parmi : l'huile d'olive, l'huile de noix de coco, l'huile de saumon, l'huile de maïs, l'huile de soja, l'huile de canola, l'huile de colza, l'huile de tournesol, l'huile de lin, l'huile de riz, l'huile de carthame, l'huile de coton, l'huile de palme, l'huile de ricin, l'huile d'arachide, l'huile de blé, l'huile de graines de citrouille, l'huile de graines de pavot, l'huile de chanvre, l'huile de pépins de grenade, l'huile de morue, l'huile de hareng, l'huile de baleine, l'huile de phoque, la margarine, le beurre, le saindoux, le suif et les mélanges de ceux-ci.

8. Composition alimentaire artificielle destinée à être utilisée selon les revendications 6 et 7, **caractérisée en ce que** le ou les ingrédients lipidiques sont choisis dans le groupe composé de : l'huile d'olive, l'huile de noix de coco et l'huile de saumon.

9. Composition alimentaire artificielle destinée à être utilisée selon une quelconque revendication précédente, **caractérisée en ce que** les glucides peuvent être choisis dans le groupe composé de : le saccharose, la cellulose, l'amidon et les mélanges de ceux-ci.

10. Composition alimentaire artificielle destinée à être utilisée selon une quelconque revendication précédente, **caractérisée en ce qu'**elle est sous une forme convenant à l'administration orale.

11. Composition alimentaire artificielle destinée à être utilisée selon la revendication 10, **caractérisée en ce qu'**elle est sous une forme solide, semi-solide ou liquide et est choisie parmi : une poudre sèche, un shake, des concentrés liquides, une boisson prête à boire, une boisson réfrigérée ou de longue conservation, une soupe, une pâte, une purée, une barre nutritionnelle.

12. Composition alimentaire artificielle destinée à être utilisée selon une quelconque revendication précédente, **caractérisée en ce que** le traitement d'un cancer comprend : un cancer du rein, un cancer du poumon, un cancer du côlon, un cancer du sein, un mélanome, un cancer de l'ovaire, un cancer de la prostate, un cancer du pancréas, un cancer du foie, un cancer de l'endomètre, un cancer du col de l'utérus, un cancer de la vessie, un cancer de l'œsophage, un cancer de l'estomac, un cancer de la tête et du cou, une leucémie, des lymphomes, des cancers de la peau autres qu'un mélanome, un sarcome, des cancers du système nerveux central, un cancer du testicule, un cancer de la thyroïde et un cancer de site primitif inconnu.

13. Composition alimentaire artificielle destinée à être utilisée selon une quelconque revendication précédente, **caractérisée en ce qu'**un tel traitement comprend la prise de la composition alimentaire artificielle en tant que remplacement d'un repas pour répondre aux besoins nutritionnels quotidiens nécessaires du sujet.

14. Composition alimentaire artificielle destinée à être utilisée selon une quelconque revendication précédente, **caractérisée en ce que** le traitement comprend des cycles de traitement de deux à douze semaines avec quatre à six doses quotidiennes.

15. Composition alimentaire artificielle destinée à être utilisée selon une quelconque revendication précédente, **caractérisée en ce qu'**elle comprend en outre de l'eau ou un vecteur à base d'eau.

16. Composition pharmaceutique comprenant la composition alimentaire artificielle destinée à être utilisée selon l'une quelconque des revendications 1 à 15 conjointement avec un vecteur ou véhicule pharmaceutiquement acceptable, destinée à être utilisée dans le traitement d'un cancer.

17. Composition pharmaceutique destinée à être utilisée selon la revendication 16, **caractérisée en ce que** le traitement d'un cancer comprend : un cancer du rein, un cancer du poumon, un cancer du côlon, un cancer du sein, un mélanome, un cancer de l'ovaire, un cancer de la prostate, un cancer du pancréas, un cancer du foie, un cancer de l'endomètre, un cancer du col de l'utérus, un cancer de la vessie, un cancer de l'œsophage, un cancer de l'estomac, un cancer de la tête et du cou, une leucémie, des lymphomes, des cancers de la peau autres qu'un mélanome, un sarcome, des cancers du système nerveux central, un cancer du testicule, un cancer de la thyroïde et un cancer de site primitif inconnu.

18. Composition pharmaceutique destinée à être utilisée selon les revendications 16 à 17, **caractérisée en ce que** le traitement comprend la co-administration de la composition pharmaceutique conjointement avec un quelconque type de thérapie médicamenteuse, notamment des médicaments de chimiothérapie cytotoxiques tels que des agents alkylants (p. ex., le cisplatine, le carboplatine, l'oxaliplatine, le cyclophosphamide, le témozolomide, l'hydroxyurée, etc.), des antimétabolites (p. ex., le fluorouracile, la capécitabine, la gemcitabine, le méthotrexate, la cytarabine, etc.), des inhibiteurs mitotiques (p. ex., le paclitaxel, le docétaxel, le cabazitaxel, la vincristine, la vinblastine, la vinorelbine, la vindésine, etc.), des inhibiteurs de topoisomérase (p. ex., l'étoposide, le téniposide, l'irinotécan, le topotécan, la doxorubicine, l'épirubicine, etc.), une hormonothérapie (p. ex. des anti-oestrogènes tels que le tamoxifène, des inhibiteurs d'aromatase tels que l'anastrozole, des agonistes de LHRH tels que la goséréline, des anti-androgènes tels que l'abiratérone et le flutamide, des corticoïdes tels que la dexaméthasone et la prednisone, etc.), des immunothérapies (p. ex., anti-PD1 telles que le nivolumab, anti-PDL1 telles que l'avélumab, anti-CTLA4 telles que l'ipilimumab, des cytokines telles que l'interleukine-2 et l'interféron, etc.), des thérapies ciblées (p. ex., des agents anti-VEGF tels que le bévacizumab, anti-VEGFR tels que le sunitinib et le sorafénib, anti-EGFR tels que le cétuximab ou l'erlotinib, anti-HER2 tels que le trastuzumab, anti-PARP tels que l'olaparib, anti-BRAF tels que le vémurafénib, etc.) et un quelconque autre médicament anticancéreux ainsi que des mélanges de ceux-ci.

19. Composition pharmaceutique destinée à être utilisée selon la revendication 18, **caractérisée en ce qu'**une telle co-administration comprend une administration séquentielle, concomitante ou simultanée de principes actifs.

20. Composition pharmaceutique destinée à être utilisée selon les revendications 16 à 19, **caractérisée en ce que** le traitement comprend l'administration à un sujet qui en a besoin d'une quantité efficace de la composition pharmaceutique pendant un traitement par chirurgie et/ou radiothérapie.
